# EUROPÄISCHE PATENTSCHRIFT

(11) **EP 0 420 808 B1**
(45) Veröffentlichungstag und Bekanntmachung des Hinweises auf die Patenterteilung: **01.03.1995**
(21) Anmeldenummer: 90810714.7
(22) Anmeldetag: 18.09.1990
(51) Int. Cl.: C07D 407/06, C07D 405/14, B41M 5/145, B41M 5/30

(54) **Pyranhaltige Phthalide**
Pyran-containing phthalides
Phtalides contenant un reste pyrannique

(30) Priorität: 27.09.1989 CH 3497/89
(43) Veröffentlichungstag der Anmeldung: 03.04.1991
(73) Patentinhaber: CIBA-GEIGY AG, 4002 Basel (CH)
(72) Erfinder: Phaff, Rox, Dr., CH-4452 Itingen (CH)

(56) Entgegenhaltungen:
- EP-A- 0 266 310

## Beschreibung

Die vorliegende Erfindung betrifft pyranhaltige Phthalide, Verfahren zu ihrer Herstellung und ihre Verwendung als Farbbildner in druckempfindlichen oder wärmeempfindlichen Aufzeichnungsmaterialien.

In der EP-A-0,266,310 werden Furangruppen aufweisende Phthalid- und Azaphthalidverbindungen beschrieben, die sich als Farbbildner in druck- und wärmeempfindlichen Aufzeichnungsmaterialien eignen.

Die erfindungsgemässen Phthalide entsprechen der allgemeinen Formel 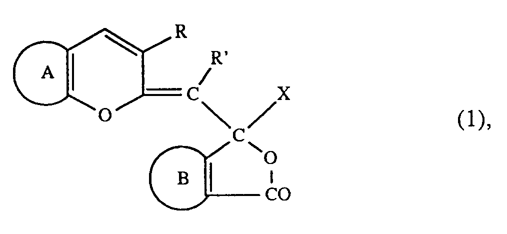
worin
R Wasserstoff, unsubstituiertes oder durch Halogen, Cyano, Hydroxy oder C₁-C₆-Alkoxy substituiertes Alkyl mit höchstens 12 Kohlenstoffatomen; Cycloalkyl; unsubstituiertes oder durch Halogen, Cyano, Methyl, Trifluormethyl, Methoxy oder Carbomethoxy substituiertes Phenyl oder Phenethyl; Phenylisopropyl, Benzyl oder einen 5- oder 6-gliedrigen heterocyclischen Rest von aromatischem Charakter; R' Wasserstoff oder R zusammen mit R' gegebenenfalls durch Methyl substituiertes C₂-C₃-Alkylen;
R₁, R₂ und R₃, unabhängig voneinander, je Wasserstoff, unsubstituiertes oder durch Halogen, Hydroxy, Cyano oder C₁-C₆-Alkoxy substituiertes Alkyl mit höchstens 12 Kohlenstoffatomen, Acyl mit 1 bis 8 Kohlenstoffatomen, Cycloalkyl mit 5 bis 10 Kohlenstoffatomen oder unsubstituiertes oder durch Halogen, Trifluormethyl, Cyano, C₁-C₆-Alkyl, C₁-C₆-Alkoxy, C₁-C₆-Alkoxycarbonyl, -NX'X'' oder X'X''N-phenylamino, ringsubstituiertes Phenethyl oder Phenyl, worin X' und X'', unabhängig voneinander, Wasserstoff, C₁-C₆-Alkyl, Cyclohexyl, Benzyl oder Phenyl darstellen, oder R₁ und R₂ zusammen mit dem sie verbindenden Stickstoffatom Pyrrolidino, Piperidino, Pipecolino, Morpholino, Thiomorpholino, Piperazino, N-Methylpiperazino, N-Phenylpiperazino oder N-Alkylimidazolino bedeuten, X Wasserstoff; Alkyl mit 1 bis 12 Kohlenstoffatomen; oder einen unsubstituierten oder durch Halogen, Cyano, C₁-C₆-Alkyl, C₅-C₆-Cycloalkyl, C₁-C₈-Acyl, -NR₁R₂, -OR₃ oder -SR₃ substituierten Phenyl- oder Naphthylrest; oder einen unsubstituierten oder durch Halogen, Hydroxy, Cyano, Nitro, C₁-C₆-Alkyl, C₁-C₆-Alkoxy, C₁-C₆-Alkylthio, C₁-C₆-Alkoxycarbonyl, Acyl mit 1 bis 8 Kohlenstoffatomen, Amino, C₁-C₆-Alkylamino, C₁-C₆-Alkylcarbonylamino oder Di-C₁-C₆-Alkylamino, C₅-C₆-Cycloalkyl, Benzyl oder Phenyl C-substituierten Thienyl-, Acridinyl-, Benzofuranyl-, Benzothienyl-, Naphthothienyl-, Phenothiazinyl-, Pyrrolyl-, Indolyl-, Carbazolyl-, Julolidinyl-, Kairolinyl-, Indolinyl-, Dihydrochinolinyl- oder Tetrahydrochinolylrest; oder einen durch C₁-C₁₂-Alkyl, C₂-C₁₂-Alkenyl, C₅-C₁₀-Cycloalkyl, C₁-C₈-Acyl, Phenyl, Benzyl, Phenethyl oder Phenisopropyl N-substituierten Acridinyl-, Phenothiazinyl-, Pyrrolyl-, Indolyl-, Carbazolyl-, Julolidinyl-, Kairolinyl-, Indolinyl-, Dihydrochinolinyl- oder Tetrahydrochinolylrest; bedeuten; und
der Ring A einen gegebenenfalls durch Halogen, C₁-C₆-Alkyl, C₁-C₆-Alkoxy oder C₁-C₆-Alkoxycarbonyl ein- oder mehrfach substituierten Benzol- oder Naphthalinring bedeuten und der Ring B einen nichtsubstituierten oder durch Halogen, Cyano, Nitro, C₁-C₆-Alkyl, C₁-C₆-Alkoxy, C₁-C₆-Alkylthio, C₁-C₆-Alkylcarbonyl, C₁-C₆-Alkoxycarbonyl, Amino, C₁-C₆-Alkylamino, Di-C₁-C₆-Alkylamino oder C₁-C₆-Alkylcarbonylamino substituierten Benzolring; oder einen Pyridin- oder Pyrazinring; oder einen Naphthalin- Chinolin- oder Chinoxalinring darstellt.

Der Ring A stellt vorteilhafterweise einen unsubstituierten oder durch Halogen oder C₁-C₆-Alkyl substituierten Benzolkern dar. Besonders bevorzugt ist ein unsubstituierter Benzolkern.

Als 6-gliedriger heteroaromatischer Ring stellt B insbesondere einen Pyridin- oder Pyrazinring dar.

Die durch B wiedergegebenen bevorzugten 6-gliedrigen aromatischen oder heteroaromatischen Reste sind der 2,3-Pyridino-, 3,4-Pyridino-, 2,3-Pyrazino-, 2,3-Chinoxalino-, 1,2-Naphthalino-, 2,3-Naphthalino- oder 1,2-Benzorest, der unsubstituiert oder durch Halogen, wie Chlor oder Brom, Nitro, C₁-C₆-Alkyl, C₁-C₆-Alkoxy, C₁-C₆-Alkylthio oder eine wie vorstehend definiert gegebenenfalls substituierte Aminogruppe substituiert wobei der unsubstituierte oder durch Chloratome, C₁-C₆-Alkoxy oder Di-C₁-C₆-Alkylamino, besonders Dimethylamino substi-Chloratome, C₁-C₆-Alkoxy oder Di-C₁-C₆-Alkylamino, besonders Dimethylamino substituierte 1,2-Benzorest besonders bevorzugt ist.

In Formel (1) ist der heteroaromatische Rest X zweckmässigerweise über ein Kohlenstoffatom des Heteroringes an das zentrale (meso) Kohlenstoffatom der Phthalidverbindung gebunden.

Der ein- oder mehrkernige heteroaromatische Rest kann einfach oder mehrfach durch C- oder N-Substituenten ringsubstituiert sein. Diese Reste können jeweils z.B. durch Cyano, Halogen, Nitro, Hydroxy, C₁-C₆-Alkyl, C₁-C₆-Alkoxy, C₁-C₆-Alkylamino oder C₁-C₆-Alkoxycarbonyl weitersubstituiert sein.

Die Alkyl- und Alkenylreste können geradkettig oder verzweigt sein. Beispiele hierfür sind Methyl, Ethyl, n-Propyl, Isopropyl, n-Butyl, 1-Methylbutyl, t-Butyl, sek.Butyl, Amyl, Isopentyl, n-Hexyl, 2-Ethyl- hexyl, Isooctyl, n-Octyl, 1,1,3,3-Tetramethylbutyl, Nonyl, Isononyl, 3-Ethylheptyl, Decyl oder n-Dodecyl bzw. Vinyl, Allyl, 2-Methylallyl, 2-Ethylallyl, 2-Butenyl oder Octenyl.

Bevorzugte heteroaromatische Reste sind z.B. N-C₁-C₈-Alkyl-pyrrol-2-yl-, N-Phenylpyrrol-3-yl-, N-C₁-C₈-Alkyl-2-methylindol-3-yl-, N-C₂-C₄-Alkanoyl-2-methylindol-3-yl, 2-Phenylindol-3-yl- oder N-C₁-C₈-Alkyl-2-phenylindol-3-ylreste.

Als aromatischer Rest stellt X vorzugsweise einen Phenylrest der Formel 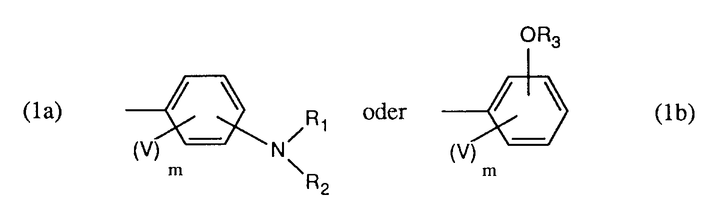
dar.

Hierbei bedeuten R₁, R₂ und R₃, unabhängig voneinander, je Wasserstoff, unsubstituiertes oder durch Halogen, Hydroxy, Cyano oder C₁-C₆-Alkoxy substituiertes Alkyl mit höchstens 12 Kohlenstoffatomen, Acyl mit 1 bis 8 Kohlenstoffatomen, Cycloalkyl mit 5 bis 10 Kohlenstoffatomen oder unsubstituiertes oder durch Halogen, Trifluormethyl, Cyano, C₁-C₆-Alkyl, C₁-C₆-Alkoxy, C₁-C₆-Alkoxycarbonyl, -NX'X'' oder X'X''N-phenylamino, ringsubstituiertes Phenalkyl oder Phenyl, worin X' und X'', unabhängig voneinander, Wasserstoff, C₁-C₆-Alkyl, Cyclohexyl, Benzyl oder Phenyl darstellen, oder R₁ und R₂ zusammen mit dem sie verbindenden Stickstoffatom einen fünf- oder sechsgliedrigen, vorzugsweise gesättigten, heterocyclischen Rest bilden. V bedeutet Wasserstoff, Halogen, C₁-C₆-Alkyl, C₁-C₁₂-Alkoxy, C₁-C₁₂-Acyloxy, Benzyl, Phenyl, Benzyloxy, Phenyloxy, durch Halogen, Cyano, C₁-C₆-Alkyl oder C₁-C₆-Alkoxy substituiertes Benzyl oder Benzyloxy oder die Gruppe -NT₁T₂. T₁ und T₂, unabhängig voneinander, bedeuten je Wasserstoff, C₁-C₆-Alkyl, C₅-C₁₀-Cycloalkyl, unsubstituiertes oder durch Halogen, Cyano, C₁-C₆-Alkyl oder C₁-C₆-Alkoxy substituiertes Benzyl, oder Acyl mit 1 bis 8 Kohlenstoffatomen und T₁ auch unsubstituiertes oder durch Halogen, Cyano, C₁-C₆-Alkyl oder C₁-C₆-Alkoxy substituiertes Phenyl. m ist 1 oder 2. -NR₁R₂ und -OR₃ befinden sich vorzugsweise in Para-Stellung zur Verknüpfungsstelle. Ein V ist vorzugsweise in Ortho-Stellung zur Verknüpfungsstelle.

Als Alkyl stellen R, X, R₁, R₂ und R₃ beispielsweise die für Alkylreste oben aufgezählten Substituenten dar.

Sind die Alkylreste in R, R₁, R₂ und R₃ substituiert, so handelt es sich vor allem um Cyanoalkyl, Halogenalkyl, Hydroxyalkyl, Alkoxyalkyl jeweils vorzugsweise mit insgesamt 2 bis 8 Kohlenstoffatomen, wie z.B. 2-Cyanoethyl, 2-Chlorethyl, 2-Hydroxyethyl, 2-Methoxyethyl, 2-Ethoxyethyl, 2,3-Dihydroxypropyl, 2-Hydroxy-3-chlorpropyl, 3-Methoxypropyl, 4-Methoxybutyl oder 4-Propoxybutyl.

Beispiele für Cycloalkyl in der Bedeutung von R, R₁, R₂, R₃, T₁ und T₂ sind Cyclopentyl, Cycloheptyl oder vorzugsweise Cyclohexyl. Die Cycloalkylreste können einen oder mehrere C₁-C₄-Alkylreste, vorzugsweise Methylgruppen, enthalten und weisen insgesamt 5 bis 10 Kohlenstoffatome auf.

Bevorzugte Substituenten in der Phenalkyl- und Phenylgruppe der R-Reste sind z.B. Halogen, Cyano, Methyl, Trifluormethyl, Methoxy oder Carbomethoxy. Beispiele für derartige araliphatische bzw. aromatische Reste sind Methylbenzyl, 2,4- oder 2,5-Dimethylbenzyl, Chlorbenzyl, Dichlorbenzyl, Cyanobenzyl, Tolyl, Xylyl, Chlorphenyl, Methoxyphenyl, 2,6-Dimethylphenyl, Trifluormethylphenyl oder Carbomethoxyphenyl.

Der Acyloxyrest in V ist beispielsweise Formyloxy, C₁-C₆-Alkylcarbonyloxy, wie z.B. Acetyloxy oder Propionyloxy, oder Benzoyloxy. Als C₁-C₁₂-Alkoxyrest kann V eine geradkettige oder verzweigte Gruppe sein, wie z.B. Methoxy, Ethoxy, Isopropoxy, n-Butoxy, tert-Butoxy, Amyloxy, 1,1,3,3-Tetramethylbutoxy, n-Hexyloxy, n-Octyloxy oder Dodecyloxy.

Wenn das Substituentenpaar (R₁ und R₂) zusammen mit dem gemeinsamen Stickstoffatom einen heterocyclischen Rest darstellt, so ist dieser beispielsweise Pyrrolidino, Piperidino, Pipecolino, Morpholino, Thiomorpholino, Piperazino, N-Alkylpiperazino, wie z.B. N-Methylpiperazino, N-Phenylpiperazino oder N-Alkylimidazolino. Bevorzugte gesättigte heterocyclische Reste für -NR₁R₂ sind Pyrrolidino, Piperidino oder Morpholino.

Die Substituenten R₁ und R₂ sind vorzugsweise Cyclohexyl, Benzyl, Phenethyl, Cyano-C₁-C₆-Alkyl z.B. β-Cyanoethyl oder in erster Linie C₁-C₆-Alkyl, wie z.B. Methyl oder Ethyl. -NR₁R₂ ist bevorzugt auch Pyrrolidinyl. R₃ ist vorzugsweise C₁-C₆-Alkyl oder Benzyl.

V kann vorteilhafterweise Wasserstoff, Halogen, C₁-C₆-Alkyl, wie z.B. Methyl, Benzyloxy, C₁-C₈-Alkoxy, in erster Linie C₁-C₆-Alkoxy, wie z.B. Methoxy, Ethoxy, Isopropoxy oder tert.Butoxy, oder die Gruppe -NT₁T₂ sein, wobei von den Resten T₁ und T₂ eines vorzugsweise C₁-C₈-Acyl oder C₁-C₆-Alkyl und das andere Wasserstoff oder C₁-C₆-Alkyl ist. Der Acylrest ist in diesem Falle besonders C₁-C₆-Alkylcarbonyl, wie z.B. Acetyl oder Propionyl. Vorzugsweise ist V Acetylamino, Dimethylamino, Diethylamino, Benzyloxy oder besonders C₁-C₆-Alkoxy und vor allem Ethoxy oder Wasserstoff.

Der Substituent X ist vorzugsweise Wasserstoff, ein N-C₁-C₈-Alkyl-2-methylindol-3-ylrest, N-C₁-C₈-Alkyl-2-phenylindol-3-ylrest oder ein substituierter Phenylrest der Formel (1a) oder (1b).

Als heterocyclischer Rest stellt R in Formel (1) zum Beispiel den Thienyl-, Furyl-, Pyrrolyl-, Pyrazolyl-, Imidazolyl- oder Pyridylrest dar.

Die bevorzugten, heterocyclischen Reste in der Bedeutung von R sind 2-Furyl, 2-Thienyl, 2-, 3- oder 4-Pyridyl oder 5-C₁-C₆-Alkoxycarbonyl-2-thienyl, wie z.B. 5-Carbomethoxy-2-thienyl.

R ist vorzugsweise C₁-C₆-Alkyl, besonders Methyl oder Phenyl. Zusammen mit R' bedeutet R z.B. 1,2-Ethylen, 1,2-Propylen, 1,3-Propylen oder 2-Methyl-1,3-propylen. 1,3-Propylenrest ist bevorzugt.

C₁-C₆-Alkyl, C₁-C₆-Alkoxy und C₁-C₆-Alkylthio stellen solche Gruppen oder Gruppenbestandteile dar, die 1 bis 6, Kohlenstoffatome aufweisen. Beispiele für derartige Gruppen sind Methyl, Ethyl, n-Propyl, Isopropyl, n-Butyl, sek.Butyl, Amyl, Isoamyl oder Hexyl bzw. Methoxy, Ethoxy, Isopropoxy, Isobutoxy, tert.Butoxy oder Amyloxy bzw. Methylthio, Ethylthio, Propylthio oder Butylthio.Insbesondere sind Alkyl-, Alkoxy- und Alkylthioreste mit 1 bis 4 Kohlenstoffatomen bevorzugt.

Halogen bedeutet beispielsweise Fluor, Brom oder vorzugsweise Chlor.

"Acyl" ist besonders Formyl, C₁-C₆-Alkylcarbonyl, wie z.B. Acetyl oder Propionyl, oder Benzoyl. Weitere Acylreste können C₁-C₆-Alkylsulfonyl, wie z.B. Methylsulfonyl oder Ethylsulfonyl sowie Phenylsulfonyl sein. Benzoyl und Phenylsulfonyl können durch Halogen, Methyl, Methoxy oder Ethoxy substituiert sein.

Besonders wichtige pyranhaltige Phthalide entsprechen der Formel 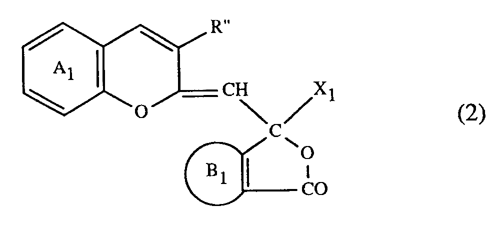
worin
der Ring A₁ unsubstituiert oder durch Halogen, C₁-C₆-Alkyl oder C₁-C₆-Alkoxy substituiert ist und der Ring B₁ einen gegebenenfalls durch Halogen, Cyano, C₁-C₆-Alkyl, C₁-C₆-Alkoxy, C₁-C₆-Alkylcarbonylamino oder Di-C₁-C₆-Alkylamino substituierten Benzol- oder Pyridinring,
R'' C₁-C₆-Alkyl, Phenyl oder durch Halogen, C₁-C₆-Alkyl oder C₁-C₆-Alkoxy substituiertes Phenyl,
X₁ Wasserstoff, einen 3-Indolylrest der Formel 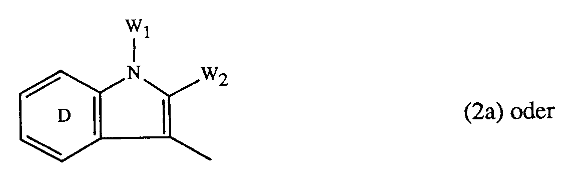
einen substituierten Phenylrest der Formel 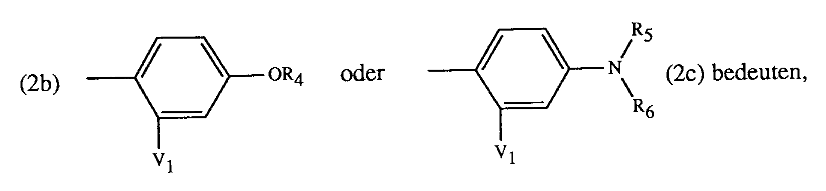
wobei W₁ Wasserstoff, unsubstituiertes oder durch Cyano oder C₁-C₆-Alkoxy substituiertes C₁-C₈-Alkyl, Acetyl, Propionyl oder Benzyl, W₂ Wasserstoff, C₁-C₆-Alkyl, vor allem Methyl, oder Phenyl,
R₄, R₅ und R₆ unabhängig voneinander, je unsubstituiertes oder durch Hydroxy, Cyano oder C₁-C₆-Alkoxy substituiertes Alkyl mit höchstens 12 Kohlenstoffatomen, C₅-C₆-Cycloalkyl, Benzyl, Phenethyl oder Phenyl, oder (R₅ und R₆) zusammen mit dem sie verbindenden Stickstoffatom Pyrrolidino, Piperidino oder Morpholino,
V₁ Wasserstoff, Halogen, C₁-C₆-Alkyl, C₁-C₈-Alkoxy, Benzyloxy oder die Gruppe -NT₃T₄,
T₃ und T₄, unabhängig voneinander, je Wasserstoff, C₁-C₆-Alkyl, C₁-C₆-Alkylcarbonyl oder unsubstituiertes oder durch Halogen, Methyl oder Methoxy substituiertes Benzoyl bedeuten, und der Ring D unsubstituiert oder durch Halogen, C₁-C₆-Alkyl, wie Methyl oder Isopropyl oder durch Di-C₁-C₆-Alkylamino, wie Dimethylamino substituiert ist.

Unter den Phthalidverbindungen der Formel (2) sind diejenigen, in denen X₁ Wasserstoff, einen 3-Indolylrest der Formel (2a), worin W₁ C₁-C₈-Alkyl, W₂ Methyl oder Phenyl bedeuten, oder einen Phenylrest der Formel (2b), in der R₄ C₁-C₆-Alkyl und V₁ Wasserstoff darstellen und der Ring B₁ einen gegebenenfalls durch C₁-C₆-Alkoxy oder Di-C₁-C₆-Alkylamino substituierten Benzolring bedeuten, bevorzugt. X₁ ist bevorzugterweise auch 4-Di-C₁-C₆-Alkylaminophenyl.

Von besonderem Interesse sind pyranhaltige Phthalidverbindungen der Formel 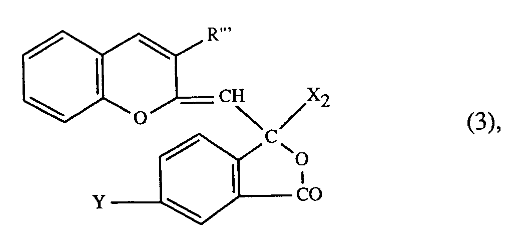
worin
- R''': C₁-C₆-Alkyl, besonders Methyl oder Phenyl,
- X₂: Wasserstoff, 1-C₁-C₈-Alkyl-2-methyl-indol-3-yl, 4-Di-C₁-C₆-Alkylaminophenyl oder 4-C₁-C₆-Alkoxyphenyl und
- Y: Wasserstoff, C₁-C₆-Alkoxy oder C₁-C₆-Alkylamino, insbesondere Dimethylamino bedeuten.

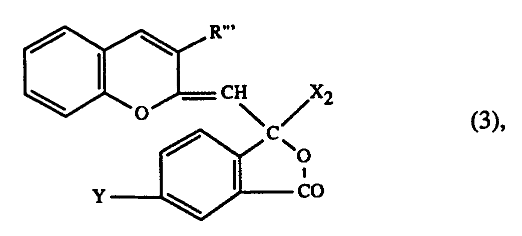

worin
- R''': Niederalkyl, besonders Methyl oder Phenyl,
- X₂: Wasserstoff, 1-C₁-C₈-Alkyl-2-methyl-indo-3-yl, 4-Diniederalkylaminophenyl oder 4-Niederalkoxyphenyl und
- Y: Wasserstoff, Niederalkoxy oder Niederalkylamino, insbesondere Dimethylamino bedeuten.

Erfindungsgemässe Verbindungen der Formel (1) können hergestellt werden, indem man ein Pyryliumsalz der Formel (4a) oder ein 1,2,3,4-Tetrahydro-Xanthyliumsalz der Formel (4b) 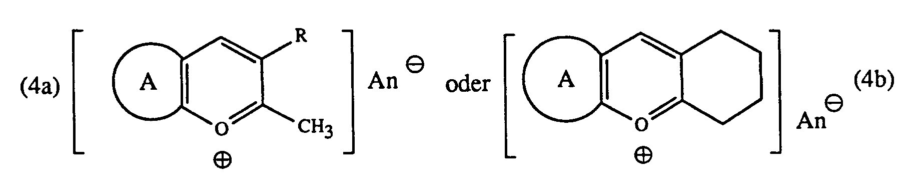
worin A und R die angegebene Bedeutung haben und
An^{⊖} ein Anion ist, mit einer Aldehydsäure bzw. Ketosäure der Formel 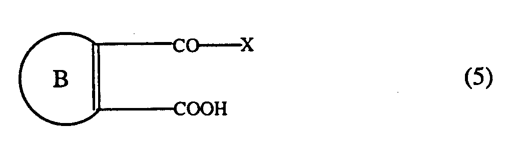
worin X und B die angegebene Bedeutung haben, umsetzt

Die Umsetzung wird zweckmässigerweise in bei der Reaktionstemperatur flüssigen organischen Lösungsmitteln z.B. Carbonsäuren wie Essigsäure, Carbonsäureanhydriden wie Essigsäureanhydrid, Acetonitril oder Propionitril, gegebenenfalls in Gegenwart saurer oder basischer Kondensationsmittel, wie z.B. Schwefelsäure, Phosphorsäure` Phosphoroxichlorid, Zinkchlorid, Toluolsulfonsäure oder Borsäure, Triethylamin, Pyridin oder Piperidin durchgeführt. Vorteilhafterweise erfolgt die Kondensation bei Temperaturen im Bereich von 20 bis 120°C, vorzugsweise 40 bis 80°C.

Der Ringschluss zum Phthalid kann zusammen mit der Kondensation oder anschliessend an diese im selben oder in einem getrennten Arbeitsgang zweckmässigerweise in Gegenwart einer Base, wie z.B. Kalium- oder Natriumhydroxid oder -carbonat, Ammoniak, aliphatischen Aminen oder Pyridin erfolgen. Die Isolierung des Endproduktes der Formel (1) erfolgt in allgemein bekannter Weise durch Abtrennen des gebildeten Niederschlages, Waschen und Trocknen oder durch Behandeln des Niederschlages mit geeigneten organischen Lösungsmitteln, wie z.B. Methanol, Isopropanol, Benzol, Chlorbenzol oder Toluol.

Die für die Herstellung der pyranhaltigen Phthalide benötigten Ausgangsprodukte der Formeln (4a) und (4b) sind zum Teil bekannt.

Die Pyryliumsalze der Formel (4a) und Xanthyliumsalze der Formel (4b) können in Form ihrer Chloride, Tetrachloroferrate, Tetrafluoroborate, Trichlorzinkate, Perborate oder Perchlorate eingesetzt werden. Bevorzugt sind Trichlorozinkate und vor allem Tetrachloroferrate.

Geeignete Pyryliumsalze bzw. Xanthyliumsalze sind:
2,3-Dimethylbenzopyrylium-tetrachloroferrat,
2-Methyl-3-phenyl-benzopyrylium-tetrachloroferrat,
2-Ethyl-3-phenyl-benzopyrylium-tetrachloroferrat,
2,3-Dimethylbenzopyrylium-trichlorozinkat,
2-Benzyl-3-phenyl-benzopyrylium-tetrachloroferrat,
2,3-Dimethyl-naphtho-2,1-b-pyrylium-tetrachloroferrat,
2-Methyl-3-phenyl-naphtho-2,1-b-pyrylium-tetrachloroferrat,
2-Benzyl-3-phenyl-naphtho-2,1-b-pyrylium-tetrachloroferrat,
1,2,3,4-Tetrahydroxanthyliumtrichlorzinkat,
1,2,3,4-Tetrahydro-benzoxanthylium-trichlorozinkat.

Die Ausgangsprodukte der Formel (5) sind grösstenteils bekannt. Zu spezifischen Beispielen gehören:
5-Methoxy-phthalaldehydsäure,
5-Dimethylaminophthalaldehydsäure,
4'-Methoxy-4-dimethylamino-benzophenon-2-carbonsäure,
4'-Diethylamino-benzophenon-2-carbonsäure,
3-(2'-Carboxybenzoyl)-1-ethyl-2-methylindol,
3-(2'-Carboxy-benzoyl)-1-n-octyl-2-methylindol,
3-(3',4',5',6'-Tetrachlor-2'-carboxy-benzoyl)-1-ethyl-2-methylindol,
2-(1'-n-Octyl-2'-methylindol-3'-yl)-carbonyl-nicotinsäure,
4,4'-Bis-dimethylamino-benzophenon-2-carbonsäure.

Die pyranhaltigen Phthalide der Formeln (1) bis (3) sind normalerweise farblos oder höchstens schwach gefärbt. Wenn diese Farbbildner mit einem vorzugsweise sauren Entwickler, d.h. einem Elektronenakzeptor, in Kontakt gebracht werden, so ergeben sie je nach der Bedeutung von X und dem verwendeten Entwickler intensive orange, rote, violette, grün-blaue, blaue oder violett-blaue Farbtöne, die sublimations- und lichtecht sind. Die Phthalide der Formeln (1) bis (3) sind auch sehr wertvoll im Gemisch mit einem oder mehreren anderen bekannten Farbbildner, z.B. 3,3-(Bis-aminophenyl-)-phthaliden, wie CVL, 3-Indolyl-3-aminophenyl- aza- oder -diazaphthaliden, (3,3-Bis-indolyl-)phthaliden, 3-Aminofluoranen, 2,6-Diaminofluoranen, 2,6-Diamino-3-methyl-fluoranen, 3,6-Bisalkoxyfluoranen, 3,6-Bisdiarylaminofluoranen, Leukoauraminen, Spiropyranen, Spirodipyranen, Chromenopyrazolen, Chromenoindolen, Phenoxazinen, Phenothiazinen, Chinazolinen, Rhodaminlaktamen, Carbazolylmethanen oder weiteren Triarylmethanleukofarbstoffen, um blaue, marine-blaue, graue oder schwarze Färbungen zu ergeben.

Die Phthalide der Formeln (1) bis (3) zeigen sowohl auf aktivierten Tonen, wie auch auf phenolischen Unterlagen eine ausgezeichnete Farbintensität und Lichtechtheit. Sie eignen sich vor allem als sich schnell entwickelnde Farbbildner für die Verwendung in einem warmeempfindlichen oder insbesondere druckempfindlichen Aufzeichnungsmaterial, das sowohl Kopier- als auch Registriermaterial sein kann. Sie zeichnen sich dadurch aus, dass sie pH stabil und in den Kapselölen hervorragend löslich sind. Nach Belichtung in CB-Blatt weisen sie eine geringe Abnahme der Farbstärke (CB-Desaktivierung) auf.

Ein druckempfindliches Material besteht beispielsweise aus mindestens einem Paar von Blättern, die mindestens einen Farbbildner der Formeln (1) bis (3) gelöst in einem organischen Lösungsmittel und einen Elektronenakzeptor als Entwickler enthalten.

Typische Beispiele für solche Entwickler sind Aktivton-Substanzen, wie Attapulgus-Ton, Säureton, Bentonit, Montmorillonit, aktivierter Ton, wie z.B. säureaktiviertes Bentonit oder Montmorillonit, ferner Zeolith, Halloysit, Siliciumdioxyd, Aluminiumoxid, Aluminiumsulfat, Aluminium- phosphat, Zinkchlorid, Zinknitrat, Zirkondioxid, aktiviertes Kaolin oder irgendein beliebiger Ton. Als Entwickler können auch sauer reagierende, organische Verbindungen, wie z.B. gegebenenfalls ringsubstituierte Phenole, Resorcine, Salicylsäuren, wie z.B. 3,5-Bis-(α,α-dimethyl- benzyl)-salicylsäure oder 3,5-Bis-(α-methylbenzyl)-salicylsäure oder Salicylsäureester und deren Metallsalze, z.B. Zinksalze, sowie ein sauer reagierendes, polymeres Material, wie z.B. ein phenolisches Polymerisat, ein Alkylphenolacetylenharz, ein Maleinsäure-Kolophonium-Harz oder ein teilweise oder vollständig hydrolysiertes Polymerisat von Maleinsäureanhydrid mit Styrol, Ethylen oder Vinylmethylether, oder Carboxymethylen verwendet werden. Es können auch Mischungen der genannten monomeren und polymeren Verbindungen eingesetzt werden. Besonders bevorzugte Entwickler sind säureaktiviertes Bentonit, Zinksalicylate oder die Kondensationsprodukte von p-substituierten Phenolen mit Formaldehyd. Die letzteren können auch mit Zink modifiziert sein. Die Zinksalicylate sind z.B. in EP-A-181 283 oder DE-A-2 242 250 beschrieben.

Die Entwickler können zusätzlich auch im Gemisch mit an sich unreäktiven oder wenig reaktiven Pigmenten oder weiteren Hilfsstoffen wie Kieselgel oder UV-Absorbern, wie z.B. 2-(2'-Hydroxyphenyl-)benztriazolen eingesetzt werden. Beispiele für solche Pigmente sind: Talk, Titandioxid, Aluminiumoxid, Aluminiumhydroxyd, Zinkoxid, Kreide, Tone wie Kaolin, sowie organische Pigmente, z.B. Harnstoff-Formaldehydkondensate (BET-Oberfläche 2-75 m²/g) oder Melamin-Formaldehyd-Kondensationsprodukte.

Der Farbbildner liefert an den Punkten, an denen er mit dem Elektronenakzeptor in Kontakt kommt, eine gefärbte Markierung. Um eine frühzeitige Aktivierung der in dem druckempfindlichen Aufzeichnungsmaterial vorhandenen Farbbildner zu verhindern, werden diese in der Regel von dem Elektronenakzeptor getrennt. Dies kann zweckmässig erzielt werden, indem man die Farbbildner in schaum-, schwamm- oder bienenwabenartigen Strukturen einarbeitet. Vorzugsweise sind die Farbbildner in Mikrokapseln eingeschlossen, die sich in der Regel durch Druck zerbrechen lassen.

Beim Zerbrechen der Kapseln durch Druck, beispielsweise mittels eines Bleistiftes, wird die Farbbildnerlösung auf ein benachbartes mit einem Elektronenakzeptor beschichtetes Blatt übertragen, wodurch eine farbige Stelle erzeugt wird. Die Farbe resultiert aus dem dabei gebildeten Farbstoff, der im sichtbaren Bereich des elektromagnetischen Spektrums absorbiert.

Die Farbbildner werden vorzugsweise in Form von Lösungen in organischen Lösungsmitteln eingekapselt. Beispiele für geeignete Lösungsmittel sind vorzugsweise nichtflüchtige Lösungsmittel, z.B. halogeniertes Paraffin, Benzol oder Diphenyl, wie Chlorparaffin, Trichlorbenzol, Monochlordiphenyl, Dichlordiphenyl oder Trichlordiphenyl, Ester, wie z.B. Tricresylphosphat, Di-n-butylphthalat, Dioctylphthalat, Trichlorethylphosphat, aromatische Ether, wie Benzylphenylether, Kohlenwasserstofföle, wie Paraffin oder Kerosin, aromatische Kohlenwasserstoffe, z.B. mit Isopropyl, Isobutyl, sek-Butyl oder tert.-Butyl alkylierte Derivate von Diphenyl, Naphthalin oder Terphenyl, Dibenzyltoluol, partiell hydriertes Terphenyl, mono- bis tetra-C₁-C₃-alkylierte Diphenylalkane, Dodecylbenzol, benzylierte Xylole, Phenylxylylethan oder weitere chlorierte oder hydrierte, kondensierte, aromatische Kohlenwasserstoffe. Oft werden Mischungen verschiedener Lösungsmittel, insbesondere Mischungen aus Paraffinölen oder Kerosin und Diisopropylnaphthalin oder partiell hydriertem Terphenyl, eingesetzt, um eine optimale Löslichkeit für die Farbbildung, eine rasche und intensive Färbung und eine für die Mikroverkapselung günstige Viskosität zu erreichen. Bei der Einkapselung zeichnen sich die erfindungsgemässen Phthalide dadurch aus, dass sie gut löslich sind und pH-Beständigkeit z.B. in einem pH-Bereich von 4 bis 10 zeigen.

Die Kapselwände können durch Koazervationskräfte gleichmässig um die Tröpfchen der Farbbildnerlösung herum gebildet werden, wobei das Einkapselungsmaterial z.B. in der US-Patentschrift 2,800,457 beschrieben ist. Die Kapseln können vorzugsweise auch aus einem Aminoplast oder modifizierten Aminoplasten durch Polykondensation gebildet werden, wie es in den britischen Patentschriften 989,264, 1,156,725, 1,301,052, 4,100,103 und 1,355,124 beschrieben ist. Ebenfalls geeignet sind Mirokapseln, welche durch Grenzflächenpolymerisation gebildet werden, wie z.B. Kapseln aus Polyester, Polycarbonat, Polysulfonamid, Polysulfonat, besonders aber aus Polyamid oder Polyurethan.

Die Farbbildner der Formeln (1) bis (3) enthaltenden Mikrokapseln können zur Herstellung von druckempfindlichen Kopiermaterialien der verschiedensten bekannten Arten verwendet werden. Die verschiedenen Systeme unterscheiden sich im wesentlichen voneinander durch die Anordnung der Kapseln, der Farbreaktanten und durch das Trägermaterial.

Bevorzugt wird eine Anordnung, bei der der eingekapselte Farbbildner in Form einer Schicht auf der Rückseite eines Uebertragungsblattes und der Elektronenakzeptor (Farbentwickler) in Form einer Schicht auf der Vorderseite eines Empfangsblattes vorhanden sind. Eine andere Anordnung der Bestandteile besteht darin, dass die den Farbbildner enthaltenden Mikrokapseln und der Entwickler in oder auf dem gleichen Blatt in Form einer oder mehrerer Einzelschichten vorliegen oder der Entwickler im Trägermaterial eingebaut ist.

Die Kapseln werden vorzugsweise mittels eines geeigneten Binders auf dem Träger befestigt. Da Papier das bevorzugte Trägermaterial ist, handelt es sich bei diesem Binder hauptsächlich um Papierbeschichtungsmittel, wie Gummi arabicum, Polyvinylalkohol, Hydroxymethylcellulose, Casein, Methylcellulose, Dextrin, Stärke, Stärkederivate oder Polymerlatices. Letztere sind beispielsweise Butadien-Styrolcopolymerisate oder Acrylhomo- oder -copolymere.

Als Papier werden nicht nur normale Papiere aus Cellulosefasern, sondern auch Papiere, in denen die Cellulosefasern (teilweise oder vollständig) durch Fasern aus synthetischen Polymerisaten ersetzt sind, verwendet. Schichtträger kann auch eine Kunststoffolie sein.

Vorzugsweise besteht das Durchschreibematerial auch darin, dass es eine kapselfreie, den Farbbildner enthaltende Schicht und eine farbentwickelnde Schicht, die als Farbentwickler mindestens ein anorganisches Metallsalz eines mehrwertigen Metalles, vor allem Halogenide oder Nitrate, wie z.B. Zinkchlorid, Zinnchlorid, Zinknitrat oder deren Gemische enthält, aufweist.

Die Verbindungen der Formeln (1) bis (3) können auch als Farbbildner in einem thermoreaktiven Aufzeichnungsmaterial verwendet werden. Dieses enthält in der Regel mindestens einen Schichtträger, einen oder mehrere Farbbildner, und einen Elektronenakzeptor und gegebenenfalls auch ein Bindemittel und/oder Wachs. Gewünschtenfalls können auch Aktivatoren oder Sensibilisatoren im Aufzeichnungsmaterial vorhanden sein.

Thermoreaktive Aufzeichnungssysteme umfassen, z.B. wärmeempfindliche Aufzeichnungs- und Kopiermaterialien und -papiere. Diese Systeme werden beispielsweise zum Aufzeichnen von Informationen, z.B. in elektronischen Rechnern, Ferndruckern, Fernschreibern oder in Aufzeichnungsgeräten und Messinstrumenten, wie z.B. Elektrocardiographen, verwendet Die Bilderzeugung (Markierung) kann auch manuell mit einer erhitzten Feder erfolgen. Eine weitere Einrichtung zur Erzeugung von Markierungen mittels Wärme sind Laserstrahlen.

Das thermoreaktive Aufzeichnungsmaterial kann so aufgebaut sein, dass der Farbbildner in einer Bindemittelschicht gelöst oder dispergiert ist und in einer zweiten Schicht der Entwickler in dem Bindemittel gelöst und dispergiert ist. Eine andere Möglichkeit besteht darin, dass sowohl der Farbbildner als auch der Entwickler in einer Schicht dispergiert sind. Die Schicht bzw. Schichten werden in spezifischen Bezirken mittels Wärme erweicht, worauf sich sofort in den erwärmten Teilen die erwünschte Farbe entwickelt.

Als Entwickler eignen sich die gleichen Elektronenakzeptoren, wie sie in druckempfindlichen Papieren verwendet werden. Beispiele für Entwickler sind die bereits erwähnten Tonminerale und Phenolharze, oder auch phenolische Verbindungen, wie sie beispielsweise in der DE-PS 1 251 348 beschrieben sind, z.B. 4-tert.Butylphenol, 4-Phenylphenol, Methylen-bis-(p-phenylphenol), 4-Hydroxydiphenylether, α-Naphthol, β-Naphthol, 4-Hydroxybenzoesäure-methylester oder -benzylester, 4-Hydroxydiphenyl-sulfon, 4'-Hydroxy-4-methyldiphenylsulfon, 4'-Hydroxy-4-isopropoxydi-phenylsulfon, 4,4'-Cyclo-hexylidendiphenol, 4,4'-Isopropylidendiphenol, 4,4'-Isopropyliden-bis-(2-methyphenol), ein Antipyrinkomplex von Zinkthiocyanat, ein Pyridinkomplex von Zinkthiocyanat, 4,4-Bis-(4-hydroxyphenyl)valeriansäure, Hydrochinon, Pyrogallol, Phloroglucin, p-, m-, o-Hydroxybenzoesäure, Hydroxyphthalsäure, Gallussäure, 1-Hydroxy-2-naphthoesäure sowie Borsäure oder organische, vorzugsweise aliphatische Dicarbonsäuren, wie z.B. Weinsäure, Oxalsäure, Maleinsäure, Zitronensäure, Citraconsäure oder Bernsteinsäure.

Vorzugsweise werden zur Herstellung des thermoreaktiven Aufzeichnungsmaterials schmelzbare, filmbildende Bindemittel verwendet. Diese Bindemittel sind normalerweise wasserlöslich, während die Phthalide und der Entwickler in Wasser schwer löslich oder unlöslich sind. Das Bindemittel sollte in der Lage sein, den Farbbildner und den Entwickler bei Raumtemperatur zu dispergieren und zu fixieren.

Bei Einwirkung von Wärme erweicht oder schmilzt das Bindemittel, so dass der Farbbildner mit dem Entwickler in Kontakt kommt und sich eine Farbe bilden kann. Wasserlösliche oder mindestens in Wasser quellbare Bindermittel sind z.B. hydrophile Polymerisate, wie Polyvinylalkohol. Polyacrylsäure, Hydroxyethylcellulose, Methylcellulose, Carboxymethylcellulose, Polyacrylamid, Polyvinylpyrrolidon, Gelatine, Stärke oder veretherte Maisstärke.

Wenn der Farbbildner und der Entwickler in zwei getrennten Schichten vorliegen, können in Wasser unlösliche Bindemittel, d.h. in nichtpolaren oder nur schwach polaren Lösungsmitteln lösliche Bindemittel, wie z.B. Naturkautschuk, synthetischer Kautschuk, chlorierter Kautschuk, Alkydharze, Polystyrol, Styrol/Butadien-Mischpolymerisate, Polymethylacrylate, Ethylcellulose, Nitrocellulose und Polyvinylcarbazol, verwendet werden. Die bevorzugte Anordnung ist jedoch diejenige, bei der der Farbbildner und der Entwickler in einer Schicht in einem wasserlöslichen Bindermittel enthalten sind.

Um die Stabilität des wärmeempfindlichen Aufzeichnungsmaterials oder die Bilddichte des entwickelten Bildes zu gewährleisten, kann das Material mit einer zusätzlichen Schutzschicht versehen sein. Derartige Schutzschichten bestehen in der Regel aus wasserlöslichen und/oder wasserunlöslichen Harzen, die herkömmliche Polymermaterialien oder wässrige Emulsionen von diesen Polymermaterialien sind.

Die thermoreaktiven Schichten und Harzschichten können weitere Zusätze enthalten. Zur Verbesserung des Weissgrades, zur Erleichterung des Bedruckens der Papiere und zur Verhinderung des Festklebens der erhitzten Feder können diese Schichten, z.B. Talk, Titandioxyd, Zinkoxyd, Aluminiumhydroxyd, Calciumcarbonat (z.B. Kreide), Tone oder auch organische Pigmente, wie z.B. Harnstoff-Formaldehydpolymerisate enthalten. Um zu bewirken, dass nur innnerhalb eines begrenzten Temperaturbereiches die Farbe gebildet wird, können Substanzen, wie Harnstoff, Thioharnstoff, Diphenylthioharnstoff, Acetamid, Acetanilid, Benzolsulfanilid, Stearinsäureamid, Bis-stearoylethylendiamid, Phthalsäureanhydrid, Metallstearate, wie z.B. Zinkstearat, Phthalsäurenitril, Dimethylterephthalat, Dibenzylterephthalat oder andere entsprechende, schmelzbare Produkte, welche das gleichzeitige Schmelzen des Farbbildners und des Entwicklers induzieren, zugesetzt werden. Bevorzugt enthalten thermographische Aufzeichnungsmaterialien Wachse, z.B. Carnaubawachs, Montanwachs, Paraffinwachs, Polyethylenwachs, Kondensate höherer Fettsäureamide und Formaldehyde und Kondensate höherer Fettsäuren und Ethylendiamin.

Eine weitere Anwendung der Verbindungen der Formeln (1) bis (3) ist die Herstellung eines Farbbildes mittels photohärtbarer Mikrokapseln, wie sie z.B. in der DE-OS 3 247 488 beschrieben sind.

In den folgenden Beispielen beziehen sich die angegebenen Prozentsätze, wenn nichts anderes angegeben ist, auf das Gewicht.

### Beispiel 1:

1,8 g 5-Methoxy-phthalaldehydsäure und 3,56 g 2,3-Dimethylbenzopyrylium-tetrachloroferrat werden in 10 ml Eisessig unter Zugabe von 0,1 ml konz. Schwefelsäure 1 Stunde bei 60°C gerührt. Nach beendeter Kondensation wird das Reaktionsprodukt abgekühlt, mit 20 ml Wasser versetzt und 30 Minuten bei Raumtemperatur gerührt, worauf der Niederschlag abfiltriert und in einem Gemisch von 300 ml Wasser und 7 ml 10 %iger Natriumhydroxidlösung gerührt wird. Die resultierende Mischung wird mit Toluol extrahiert. Die Toluolphase wird abgetrennt, mit Aktivkohle gereinigt, über Natriumsulfat getrocknet und eingedampft. Der Rückstand wird an Kieselgel chromatographiert. Man erhält 1,44 g der Verbindung der Formel 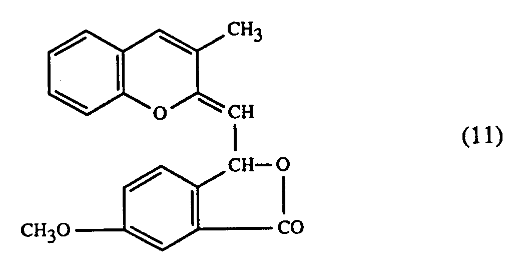
Der Schmelzpunkt dieser Verbindung liegt bei 97-99°C (Zers.). Auf saurem Ton ergibt diese Verbindung ein lichtechtes rotes Bild.

### Beispiel 2:

Verwendet man in Beispiel 1 anstelle von 5-Methoxyphthalaldehydsäure 1,93 g von 5-Dimethylaminophthalaldehydsäure und verfährt ansonst wie in Beispiel 1 beschrieben, erhält man 1,47 g einer Verbindung der Formel 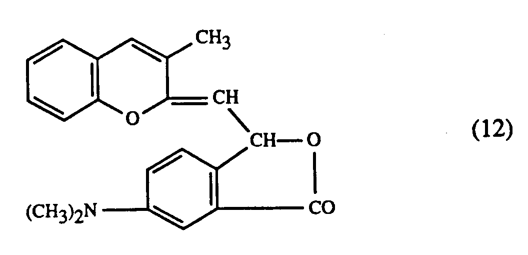
Der Schmelzpunkt dieser Verbindung liegt bei 141-142°C. Auf aktiviertem Ton entwickelt diese Verbindung ein lichtechtes blaues Bild

### Beispiel 3:

2,99 g 4'-Methoxy-4-dimethylamin-benzophenon-2-carbonsäure und 3,57 g 2,3-Dimethylbenzopyrylium-tetrachloroferrat werden in 20 ml Essigsäureanhydrid 7 Stunden bei Raumtemperatur gerührt. Nach beendeter Kondensation wird das Reaktionsprodukt auf Eis gegossen, mit Natriumhydroxidlösung basisch gestellt, mit Toluol behandelt und filtriert. Die Phasen des Filtrates werden getrennt, worauf die organische Phase mit Aktivkohle behandelt, über Natriumsulfat getrocknet und eingedampft wird. Die Säulenchromatographie des Rückstandes ergibt 2,49 g einer Verbindung der Formel 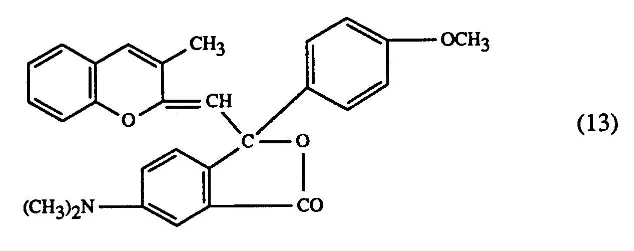
Der Schmelzpunkt dieser Verbindung liegt bei 112-113°C. Auf säuremodifiziertem Ton entwickelt diese Verbindung eine lichtechte, graublaue Farbe.

### Beispiel 4:

Verwendet man in Beispiel 3 anstelle der 4'-Methoxy-4-dimethylaminobenzophenon-2-carbonsäure 3,07 g 3-(2'-Carboxybenzoyl)-1-ethyl-2-methylindol und verfährt ansonst wie in Beispiel 3 beschrieben, erhält man eine Verbindung der Formel 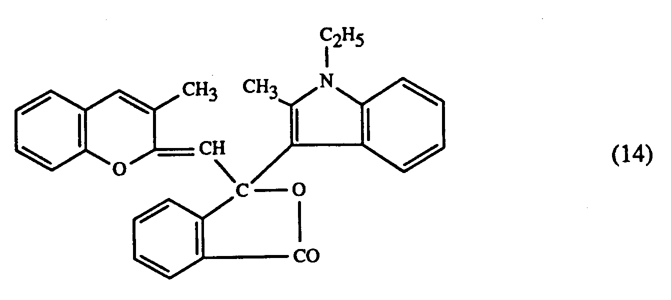
Der Schmelzpunkt dieser Verbindung liegt bei 193- 195°C. In Kontakt mit säuremodifiziertem Ton entwickelt diese Verbindung eine intensive grünstichige Blaufärbung.

Auf gleiche Art und Weise wie in Beispiel 3 beschrieben, erhält man unter Verwendung der entsprechenden Ausgangsstoffe erfindungsgemässe Phthalide der nachfolgenden Formeln, welche nachstehende Schmelzpunkte aufweisen und Farben entwickeln.

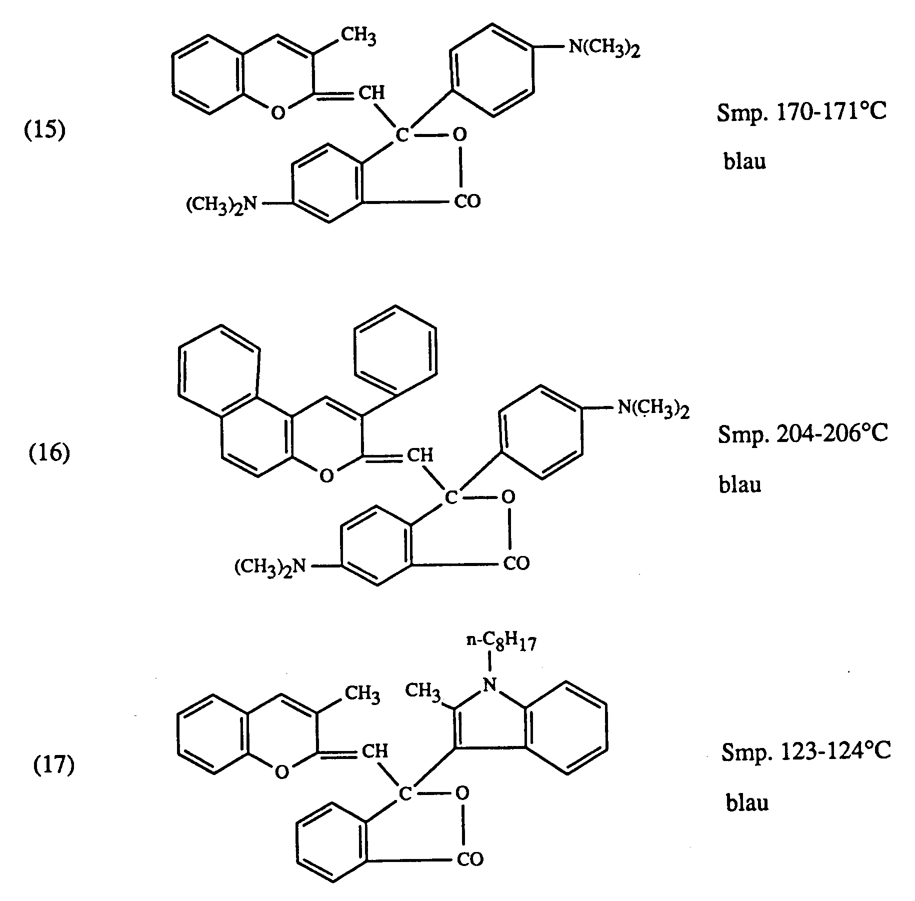

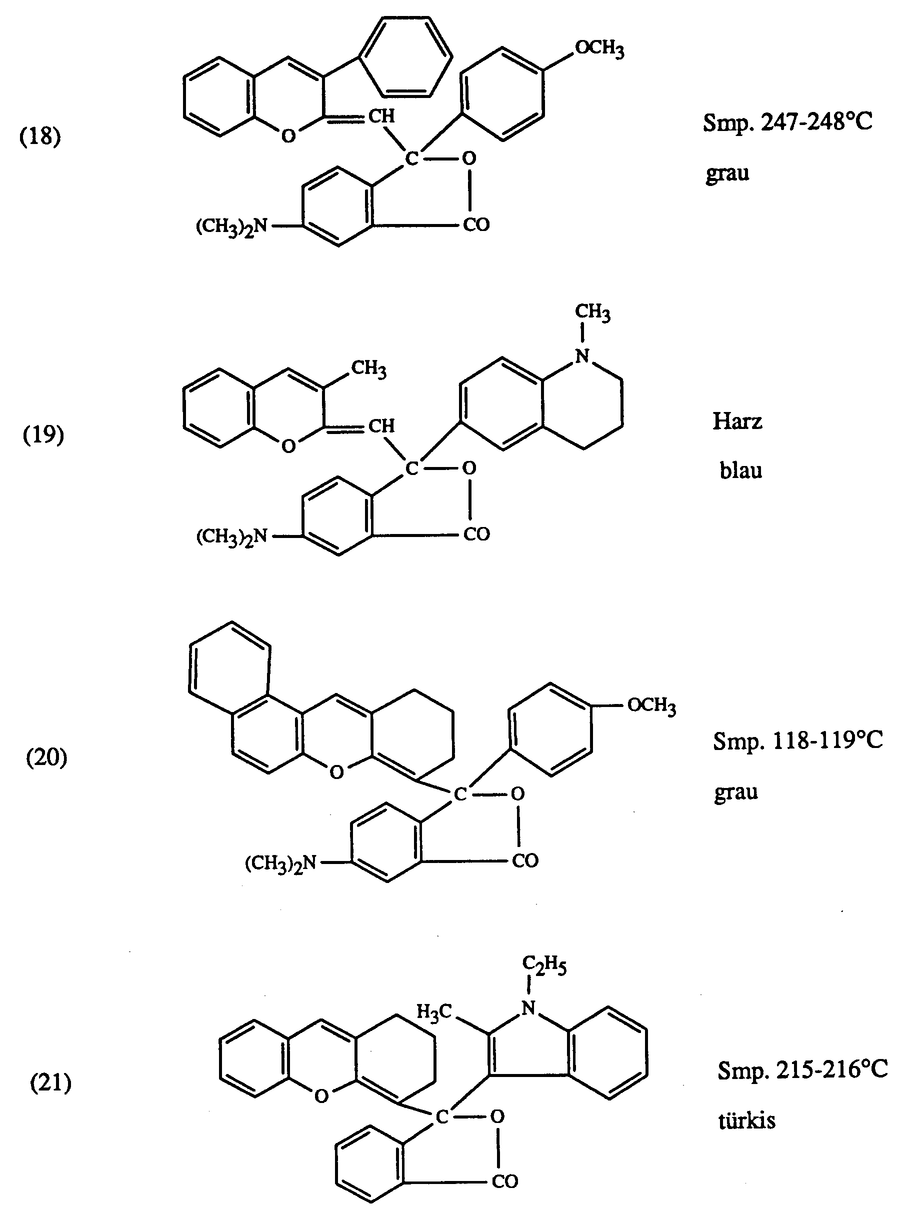

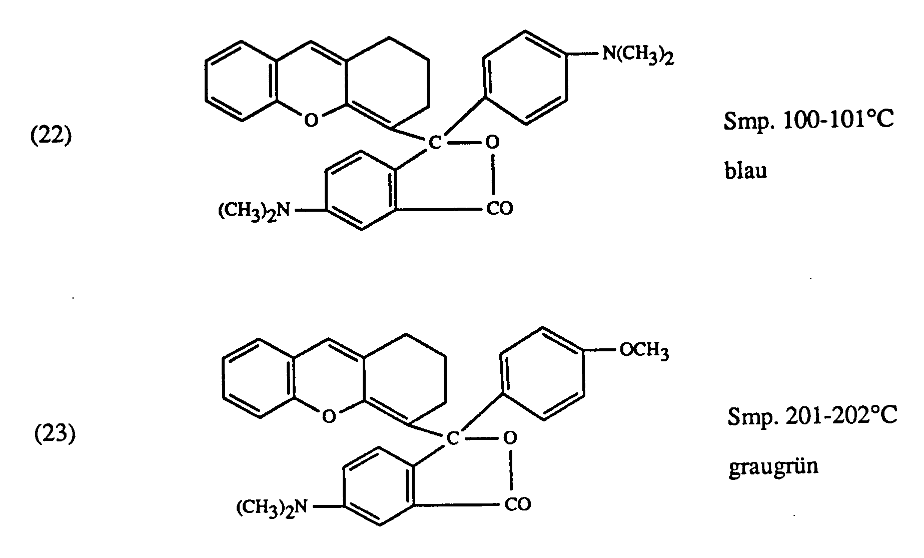

### Beispiel 5: Herstellung eines druckempfindlichen Kopierpapiers.

Eine Lösung von 3 g des pyranhaltigen Phthalides der Formel (13) (Beispiel 3) in 80 g Diisopropylnaphthalin und 17 g Kerosin wird auf an sich bekannte Weise mit Gelatine und Gummi arabicum durch Koazervation mikroverkapselt, mit Stärkelösung vermischt und auf ein Blatt Papier gestrichen. Ein zweites Blatt Papier wird auf der Frontseite mit Aktivton als Farbentwickler beschichtet. Das erste den Farbbildner enthaltende Blatt und das mit Farbentwickler beschichtete Papier werden mit den Beschichtungen benachbart aufeinandergelegt. Durch Schreiben mit der Hand oder mit der Schreibmaschine auf dem ersten Blatt wird Druck ausgeübt, und es entwickelt sich sofort auf dem mit dem Entwickler beschichteten Blatt eine intensive blaue Kopie, die ausgezeichnet lichtecht ist.

Eine entsprechende intensive, lichtechte Kopie wird auch bei Verwendung jedes der anderen in den Herstellungs-Beispielen angegebenen Farbbildner der Beispiele 1,2 und 4 erzielt.

### Beispiel 6:

1 g des pyranhaltigen Phthalides der Formel (11) gemäss Beispiel 1 wird in 17 g Toluol gelöst. Zu dieser Lösung gibt man unter Rühren 12 g Polyvinylacetat, 8 g Calciumcarbonat und 2 g Titandioxid. Die erhaltene Suspension wird im Gewichtsverhältnis 1:1 mit Toluol verdünnt und mit einem 10 »m Rakel auf ein Blatt Papier gestrichen. Auf dieses Blatt Papier wird ein zweites Blatt Papier gelegt, dessen Unterseite bei einem Auftragsgewicht von 3 g/m² mit einer Mischung bestehend aus 1 Teil eines Amidwachses, 1 Teil eines Stearinwachses und 1 Teil Zinkchlorid beschichtet ist. Durch Schreiben mit der Hand oder mit der Schreibmaschine auf dem oberen Blatt wird Druck ausgeübt, und es entwickelt sich sofort auf dem mit dem Farbbildner beschichteten Blatt eine intensive und lichtechte rote Farbe.

### Beispiel 7: Herstellung eines wärmeempfindlichen Aufzeichnungsmaterials

In einer Kugelmühle werden 32 g 4,4'-Isopropylidendiphenol (Bisphenol A), 3,8 g Distearylamid des Ethylendiamins, 39 g Kaolin, 20 g eines zu 88 % hydrolysierten Polyvinylalkohols und 500 ml Wasser gemahlen bis die Teilchengrösse ca. 5 »m beträgt. In einer zweiten Kugelmühle werden 6 g des pyranhaltigen Phthalides gemäss Beispiel 3, 3 g eines zu 88 % hydrolysierten Polyvinylalkohols und 60 ml Wasser zu einer Teilchengrösse von ca. 3 »m gemahlen.

Die beiden Dispersionen werden zusammengegeben und mit einem Trockenauftragsgewicht von 5,5 g/m² auf ein Papier gestrichen. Durch Berührung des Papiers mit einem erhitzten Kugelschreiber wird eine intensive blaue Farbe erhalten, die eine ausgezeichnete Licht- und Sublimierechtheit hat.

Intensive und lichtechte Farben können auch bei Verwendung jedes der anderen Farbbildner gemäss Beispielen 1,2 und 4 erhalten werden.

### Beispiel 8: 1 g des Farbbildners der Formel

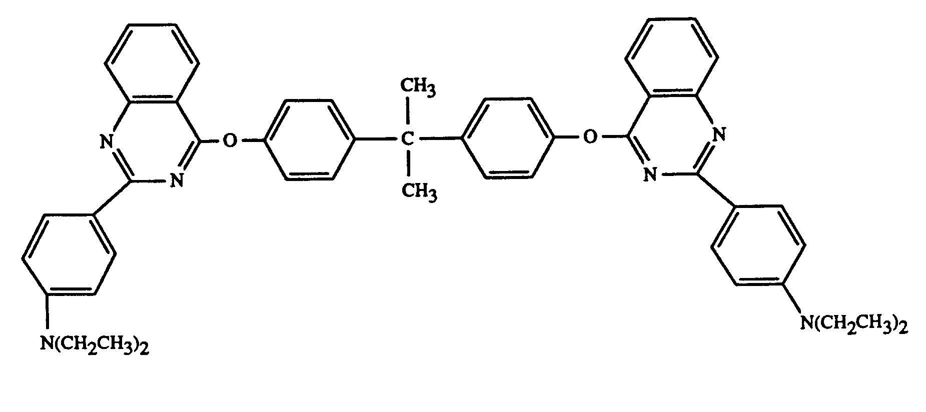

2,65 g 3,3-Bis-(1'-n-octyl-2'-methylindol-3'-yl)phthalid und
1,35 g des pyranhaltigen Phthalides der Formel (15) werden in 100 g partiell hydriertem Terphenyl bei 70-80°C gelöst. Die abgekühlte Lösung wird mit einem Tiefdruckgerät auf ein vorgeleimtes und mit aktiviertem Clay beschichtetes Papier gebracht. Es entwickelt sich sofort eine intensive und lichtechte Schwarzfärbung.

## Patentansprüche

1. Pyranhaltige Phthalide der Formel 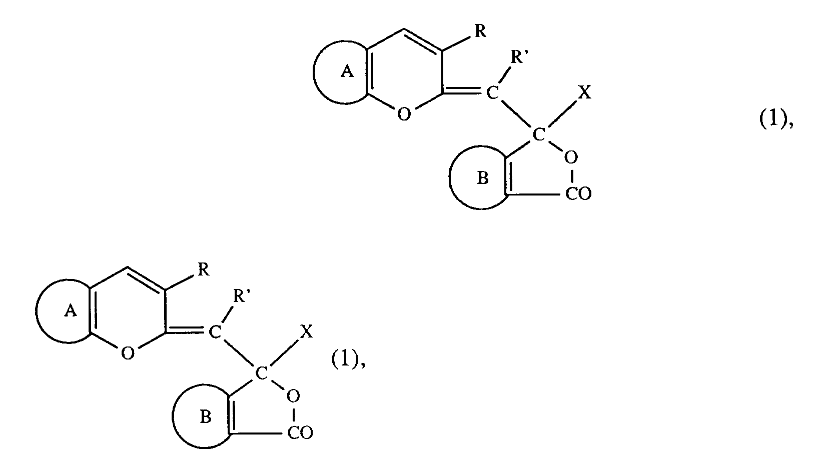 worin
R Wasserstoff, unsubstituiertes oder durch Halogen, Cyano, Hydroxy oder C₁-C₆-Alkoxy substituiertes Alkyl mit höchstens 12 Kohlenstoffatomen; Cycloalkyl; unsubstituiertes oder durch Halogen, Cyano, Methyl, Trifluormethyl, Methoxy oder Carbomethoxy substituiertes Phenyl oder Phenethyl; Phenylisopropyl, Benzyl oder einen 5- oder 6-gliedrigen heterocyclischen Rest von aromatischem Charakter; R' Wasserstoff oder R zusammen mit R' gegebenenfalls durch Methyl substituiertes C₂-C₃-Alkylen;
R₁, R₂ und R₃, unabhängig voneinander, je Wasserstoff, unsubstituiertes oder durch Halogen, Hydroxy, Cyano oder C₁-C₆-Alkoxy substituiertes Alkyl mit höchstens 12 Kohlenstoffatomen, Acyl mit 1 bis 8 Kohlenstoffatomen, Cycloalkyl mit 5 bis 10 Kohlenstoffatomen oder unsubstituiertes oder durch Halogen, Trifluormethyl, Cyano, C₁-C₆-Alkyl, C₁-C₆-Alkoxy, C₁-C₆-Alkoxycarbonyl, -NX'X'' oder X'X''N-phenylamino, ringsubstituiertes Phenethyl oder Phenyl, worin X' und X'', unabhängig voneinander, Wasserstoff, C₁-C₆-Alkyl, Cyclohexyl, Benzyl oder Phenyl darstellen, oder R₁ und R₂ zusammen mit dem sie verbindenden Stickstoffatom Pyrrolidino, Piperidino, Pipecolino, Morpholino, Thiomorpholino, Piperazino, N-Methylpiperazino, N-Phenylpiperazino oder N-Alkylimidazolino;
X Wasserstoff; Alkyl mit 1 bis 12 Kohlenstoffatomen; oder einen unsubstituierten oder durch Halogen, Cyano, C₁-C₆-Alkyl, C₅-C₆-Cycloalkyl, C₁-C₈-Acyl, -NR₁R₂, -OR₃ oder -SR₃ substituierten Phenyl- oder Naphthylrest; oder einen unsubstituierten oder durch Halogen, Hydroxy, Cyano, Nitro, C₁-C₆-Alkyl, C₁-C₆-Alkoxy, C₁-C₆-Alkylthio, C₁-C₆-Alkoxycarbonyl, Acyl mit 1 bis 8 Kohlenstoffatomen, Amino, C₁-C₆-Alkylamino, C₁-C₆-Alkylcarbonylamino oder Di-C₁-C₆-Alkylamino, C₅-C₆-Cycloalkyl, Benzyl oder Phenyl C-substituierten Thienyl-, Acridinyl-, Benzofuranyl-, Benzothienyl-, Naphthothienyl-, Phenothiazinyl-, Pyrrolyl-, Indolyl-, Carbazolyl-, Julolidinyl-, Kairolinyl-, Indolinyl-, Dihydrochinolinyl- oder Tetrahydrochinolylrest; oder einen durch C₁-C₁₂-Alkyl, C₂-C₁₂-Alkenyl, C₅-C₁₀-Cycloalkyl, C₁-C₈-Acyl, Phenyl, Benzyl, Phenethyl oder Phenisopropyl N-substituierten Acridinyl-, Phenothiazinyl-, Pyrrolyl-, Indolyl-, Carbazolyl-, Julolidinyl-, Kairolinyl-, Indolinyl-, Dihydrochinolinyl- oder Tetrahydrochinolylrest; bedeuten; und
der Ring A einen gegebenenfalls durch Halogen, C₁-C₆-Alkyl, C₁-C₆-Alkoxy oder C₁-C₆-Alkoxycarbonyl ein- oder mehrfach substituierten Benzol- oder Naphthalinring bedeuten und der Ring B einen nichtsubstituierten oder durch Halogen, Cyano, Nitro, C₁-C₆-Alkyl, C₁-C₆-Alkoxy, C₁-C₆-Alkylthio, C₁-C₆-Alkylcarbonyl, C₁-C₆-Alkoxycarbonyl, Amino, C₁-C₆-Alkylamino, Di-C₁-C₆-Alkylamino oder C₁-C₆-Alkylcarbonylamino substituierten Benzolring; oder einen Pyridin- oder Pyrazinring; oder einen Naphthalin-, Chinolin- oder Chinoxalinring darstellt.

2. Phthalide gemäss Anspruch 1, dadurch gekennzeichnet, dass in Formel (1) der Ring A einen unsubstituierten oder durch Halogen oder C₁-C₆-Alkyl substituierten Benzolkern darstellt.

3. Phthalide gemäss einem der Ansprüche 1 und 2, dadurch gekennzeichnet, dass der Ring B einen gegebenenfalls substituierten Benzol-, Naphthalin-, Pyridin-, Pyrazin-, Chinoxalin- oder Chinolinring darstellt.

4. Phthalide gemäss einem der Ansprüche 1 bis 3, dadurch gekennzeichnet, dass in Formel (1) der Ring B einen unsubstituierten oder durch Chlor, C₁-C₆-Alkoxy oder Di-C₁-C₆-Alkylamino substituierten Benzolring darstellt.

5. Phthalide gemäss einem der Ansprüche 1 bis 4, dadurch gekennzeichnet, dass in Formel (1) X einen substituierten Phenylrest der Formel 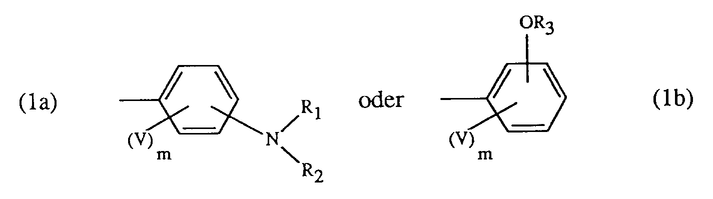 darstellt, in denen R₁, R₂ und R₃, unabhängig voneinander, je Wasserstoff, unsubstituiertes oder durch Halogen, Hydroxy, Cyano oder C₁-C₆-Alkoxy substituiertes Alkyl mit höchstens 12 Kohlenstoffatomen, Acyl mit 1 bis 8 Kohlenstoffatomen, Cycloalkyl mit 5 bis 10 Kohlenstoffatomen oder unsubstituiertes oder durch Halogen, Trifluormethyl, Cyano, C₁-C₆-Alkyl, C₁-C₆-Alkoxy, C₁-C₆-Alkoxycarbonyl, -NX'X'' oder X'X''N-phenylamino, ringsubstituiertes Phenalkyl oder Phenyl, worin X' und X'', unabhängig voneinander, Wasserstoff, C₁-C₆-Alkyl, Cyclohexyl, Benzyl oder Phenyl darstellen, oder R₁ und R₂ zusammen mit dem sie verbindenden Stickstoffatom einen fünf- oder sechsgliedrigen, heterocyclischen Rest bedeuten,
V Wasserstoff, Halogen, C₁-C₆-Alkyl, C₁-C₁₂-Alkoxy, C₁-C₁₂-Acyloxy, Benzyl, Phenyl, Benzyloxy, Phenyloxy, durch Halogen, Cyano, C₁-C₆-Alkyl oder C₁-C₆-Alkoxy substituiertes Benzyl oder Benzyloxy oder die Gruppe -NT₁T₂ bedeutet, wobei T₁ und T₂, unabhängig voneinander, je Wasserstoff, C₁-C₆-Alkyl, C₅-C₁₀Cycloalkyl, unsubstituiertes oder durch Halogen, Cyano, C₁-C₆-Alkyl oder C₁-C₆-Alkoxy substituiertes Benzyl, oder Acyl mit 1 bis 8 Kohlenstoffatomen und T₁ auch unsubstituiertes oder durch Halogen, Cyano, C₁-C₆-Alkyl oder C₁-C₆-Alkoxy substituiertes Phenyl bedeuten und m 1 oder 2 ist.

6. Phthalide gemäss einem der Ansprüche 1 bis 5, dadurch gekennzeichnet, dass in Formel (1) X Wasserstoff, einen N-C₁-C₈-Alkyl-2-methylindol-3-ylrest, einen N-C₁-C₈-Alkyl-2-phenylindol-3-ylrest oder einen substituierten Phenylrest der Formel (1a) oder (1b) bedeutet.

7. Phthalide gemäss einem der Ansprüche 1 bis 6, dadurch gekennzeichnet, dass in Formel (1) R C₁-C₆-Alkyl oder Phenyl bedeutet.

8. Phthalide gemäss einem der Ansprüche 1 bis 6, dadurch gekennzeichnet, dass in Formel (1) R zusammen mit R' gegebenenfalls durch Methyl substituiertes C₂-C₃-Alkylen bedeutet.

9. Phthalide gemäss Anspruch 1 dadurch gekennzeichnet, dass sie der Formel 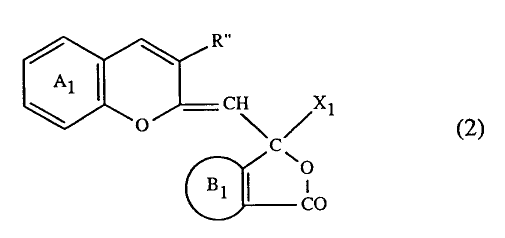 entsprechen, worin
der Ring A₁ unsubstituiert oder durch Halogen, C₁-C₆-Alkyl oder C₁-C₆-Alkoxy substituiert ist und der Ring B₁ einen gegebenenfalls durch Halogen, Cyano, C₁-C₆-Alkyl, C₁-C₆-Alkoxy, C₁-C₆-Alkylcarbonylamino oder Di-C₁-C₆-Alkylamino substituierten Benzol- oder Pyridinring,
R'' C₁-C₆-Alkyl, Phenyl oder durch Halogen, C₁-C₆-Alkyl oder C₁-C₆-Alkoxy substituiertes Phenyl,
X₁ Wasserstoff, einen 3-Indolylrest der Formel 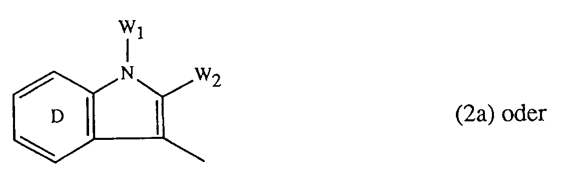 einen Phenylrest der Formel 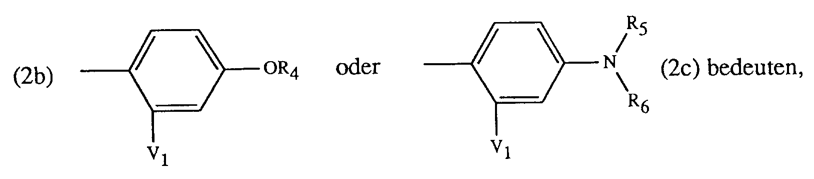 wobei W₁ Wasserstoff, unsubstituiertes oder durch Cyano oder C₁-C₆-Alkoxy substituiertes C₁-C₈-Alkyl, Acetyl, Propionyl oder Benzyl, W₂ Wasserstoff, C₁-C₆-Alkyl oder Phenyl,
R₄, R₅ und R₆ unabhängig voneinander, je unsubstituiertes oder durch Hydroxy, Cyano oder C₁-C₆-Alkoxy substituiertes Alkyl mit höchstens 12 Kohlenstoffatomen, C₅-C₆-Cycloalkyl, Benzyl, Phenethyl oder Phenyl, oder (R₅ und R₆) zusammen mit dem sie verbindenden Stickstoffatom Pyrrolidino, Piperidino oder Morpholino,
V₁ Wasserstoff, Halogen, C₁-C₆-Alkyl, C₁-C₈-Alkoxy, Benzyloxy oder die Gruppe -NT₃T₄,
T₃ und T₄, unabhängig voneinander, je Wasserstoff, C₁-C₆-Alkyl, C₁-C₆-Alkylcarbonyl oder unsubstituiertes oder durch Halogen, Methyl oder Methoxy substituiertes Benzoyl bedeuten, und der Ring D unsubstituiert oder durch Halogen, C₁-C₆-Alkyl, oder Di-C₁-C₆-Alkylamino substituiert ist.

10. Phthalide gemäss Anspruch 9, dadurch gekennzeichnet, dass in Formel (2) X₁ Wasserstoff, einen 3-Indolylrest der Formel (2a), in der W₁ C₁-C₈-Alkyl, W₂ Methyl oder Phenyl bedeutet, oder
einen Phenylrest der Formel (2b), in der R₄ C₁-C₆-Alkyl und V₁ Wasserstoff darstellen, und
der Ring B₁ einen gegebenenfalls durch C₁-C₆-Alkoxy oder Di-C₁-C₆-Alkylamino substituierten Benzolring bedeuten.

11. Phthalide gemäss Anspruch 1 dadurch gekennzeichnet, dass sie der Formel 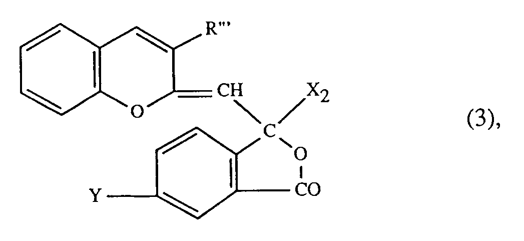 entsprechen, worin
R''' C₁-C₆-Alkyl oder Phenyl,
X₂ Wasserstoff, 4-Di-C₁-C₆-Alkylaminophenyl, 4-C₁-C₆-Alkoxyphenyl oder 1-C₁-C₈-Alkyl-2-methyl-indol-3-yl und
Y Wasserstoff, C₁-C₆-Alkoxy oder Di-C₁-C₆-Alkylamino bedeuten.

12. Phthalide gemäss Anspruch 11, dadurch gekennzeichnet, dass in Formel (3) Y Dimethylamino bedeutet.

13. Verfahren zur Herstellung von pyranhaltigen Phthaliden gemäss Anspruch 1, dadurch gekennzeichnet, dass man ein Pyryliumsalz der Formel (4a) oder ein 1,2,3,4-Tetrahydro-Xanthyliumsalz der Formel (4b) 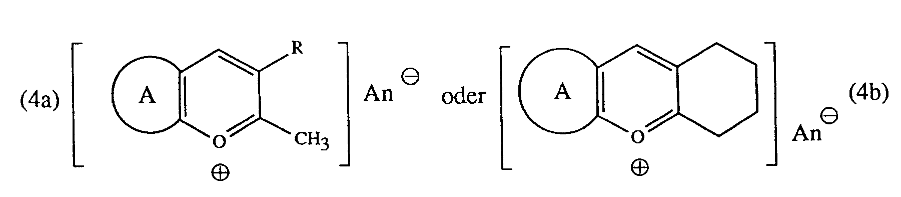 worin A und R die in Anspruch 1 angegebene Bedeutung haben und An^{⊖} ein Anion ist, mit einer Aldehydsäure bzw. Ketosäure der Formel 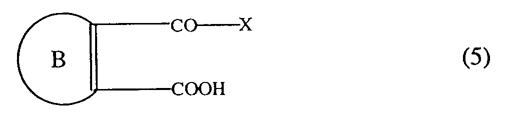 worin X und B die in Anspruch 1 angegebene Bedeutung haben, umsetzt.

14. Druck- oder wärmeempfindliches Aufzeichnungsmaterial, dadurch gekennzeichnet, dass es in seinem Farbreaktantensystem als Farbbildner mindestens eine Phthalidverbindung gemäss einem der Ansprüche 1 bis 12 enthält.

15. Druckempfindliches Aufzeichnungsmaterial gemäss Anspruch 14, dadurch gekennzeichnet, dass es die Phthalidverbindung, gelöst in einem organischen Lösungsmittel, und mindestens einen festen Elektronenakzeptor enthält.

16. Druckempfindliches Aufzeichnungsmaterial gemäss einem der Ansprüche 14 und 15, dadurch gekennzeichnet, dass die Phthalidverbindung in Mikrokapseln eingekapselt ist.

17. Druckempfindliches Aufzeichnungsmaterial gemäss Anspruch 16, dadurch gekennzeichnet, dass die eingekapselte Phthalidverbindung in Form einer Schicht auf der Rückseite eines Übertragungsblattes und der Elektronenakzeptor in Form einer Schicht auf der Vorderseite des Empfangsblattes vorhanden sind.

18. Wärmeempfindliches Aufzeichnungsmaterial gemäss Anspruch 14, dadurch gekennzeichnet, dass es in mindestens einer Schicht mindestens eine Phthalidverbindung gemäss einem der Ansprüche 1 bis 14, einen Elektronenakzeptor und gegebenenfalls ein Bindemittel und/oder Wachs enthält.

19. Aufzeichnungsmaterial gemäss einem der Ansprüche 14 bis 18, dadurch gekennzeichnet, dass die Phthalidverbindung gemeinsam mit einem oder mehreren anderen Farbbildnern enthalten ist.

## Claims

1. A pyran-containing phthalide of the formula 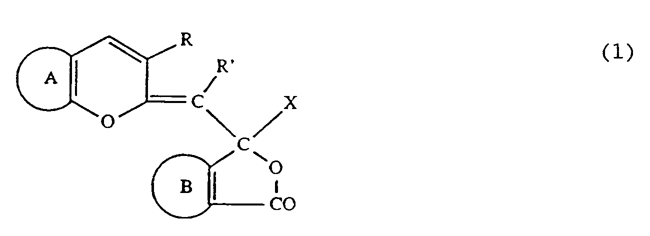 in which
R is hydrogen, alkyl which has not more than 12 carbon atoms and is unsubstituted or substituted by halogen, cyano, hydroxyl or C₁-C₆alkoxy; cycloalkyl; phenyl or phenethyl each of which is unsubstituted or substituted by halogen, cyano, methyl, trifluoromethyl, methoxy or carbomethoxy; phenylisopropyl, benzyl or a 5- or 6-membered heterocyclic radical of aromatic character; R' is hydrogen or R, together with R', is C₂-C₃alkylene which is unsubstituted or substituted by methyl; R₁, R₂ and R₃ independently of one another are each hydrogen, alkyl which has not more than 12 carbon atoms and is unsubstituted or substituted by halogen, hydroxyl, cyano or C₁-C₆alkoxy, acyl having 1 to 8 carbon atoms, cycloalkyl having 5 to 10 carbon atoms, or phenethyl or phenyl each of which is unsubstituted or ring-substituted by halogen, trifluoromethyl, cyano, C₁-C₆alkyl, C₁-C₆alkoxy, C₁-C₆alkoxycarbonyl, -NX'X'' or X'X''N-phenylamino in which X' and X'' independently of one another are hydrogen, C₁-C₆alkyl, cyclohexyl, benzyl or phenyl, or R₁ and R₂, together with the nitrogen atom linking them, are pyrrolidino, piperidino, pipecolino, morpholino, thiomorpholino, piperazino, N-methylpiperazino, N-phenylpiperazino or N-alkylimidazolino;
X is hydrogen, alkyl having 1 to 12 carbon atoms or a phenyl or naphthyl radical each of which is unsubstituted or substituted by halogen, cyano, C₁-C₆alkyl, C₅-C₆cycloalkyl, C₁-C₈acyl, -NR₁R₂, -OR₃ or -SR₃; or a thienyl, acridinyl, benzofuranyl, benzothienyl, naphthothienyl, phenothiazinyl, pyrrolyl, indolyl, carbazolyl, julolidinyl, kairolinyl, indolinyl, dihydroquinolinyl or tetrahydroquinolyl radical each of which is unsubstituted or C-substituted by halogen, hydroxyl, cyano, nitro, C₁-C₆alkyl, C₁-C₆alkoxy, C₁-C₆alkylthio, C₁-C₆alkoxycarbonyl, acyl having 1 to 8 carbon atoms, amino, C₁-C₆alkylamino, C₁-C₆alkylcarbonylamino or di-C₁-C₆alkylamino, C₅-C₆cycloalkyl, benzyl or phenyl; or an acridinyl, phenothiazinyl, pyrrolyl, indolyl, carbazolyl, julolidinyl, kairolinyl, indolinyl, dihydroquinolinyl or tetrahydroquinolyl radical each of which is N-substituted by C₁-C₁₂alkyl, C₂-C₁₂alkenyl, C₅-C₁₀cycloalkyl, C₁-C₈acyl, phenyl, benzyl, phenethyl or phenisopropyl; and
the ring A is a benzene or naphthalene ring which is unsubstituted or monosubstituted or polysubstituted by halogen, C₁-C₆alkyl, C₁-C₆alkoxy or C₁-C₆alkoxycarbonyl, and the ring B is a benzene ring which is unsubstituted or substituted by halogen, cyano, nitro, C₁-C₆alkyl, C₁-C₆alkoxy, C₁-C₆alkylthio, C₁-C₆alkylcarbonyl, C₁-C₆alkoxycarbonyl, amino, C₁-C₆alkylamino, di-C₁-C₆alkylamino or C₁-C₆alkylcarbonylamino; or a pyridine or pyrazine ring; or a naphthalene, quinoline or quinoxaline ring.

2. A phthalide according to claim 1, wherein the ring A in formula (1) is a benzene nucleus which is unsubstituted or substituted by halogen or C₁-C₆alkyl.

3. A phthalide according to either claim 1 or claim 2, wherein the ring B is an unsubstituted or substituted benzene, naphthalene, pyridine, pyrazine, quinoxaline or quinoline ring.

4. A phthalide according to any one of claims 1 to 3, wherein the ring B in formula (1) is a benzene ring which is unsubstituted or substituted by chlorine, C₁-C₆alkoxy or di-C₁-C₆alkylamino.

5. A phthalide according to any one of claims 1 to 4, wherein X in formula (1) is a substituted phenyl radical of the formula 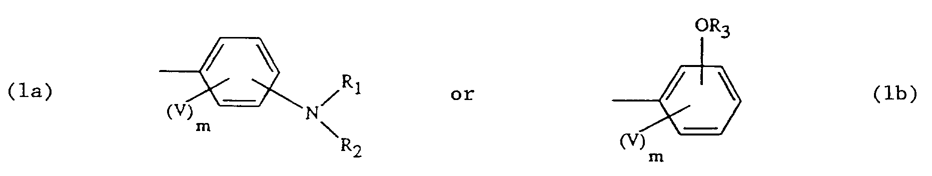 in which R₁, R₂ and R₃ independently of one another are each hydrogen, alkyl which has not more than 12 carbon atoms and is unsubstituted or substituted by halogen, hydroxyl, cyano or C₁-C₆alkoxy, acyl having 1 to 8 carbon atoms or cycloalkyl having 5 to 10 carbon atoms, or phenalkyl or phenyl each of which is unsubstituted or ring-substituted by halogen, trifluoromethyl, cyano, C₁-C₆alkyl, C₁-C₆alkoxy, C₁-C₆alkoxycarbonyl, -NX'X'' or X'X''N-phenylamino in which X' and X'' independently of one another are hydrogen, C₁-C₆alkyl, cyclohexyl, benzyl or phenyl, or R₁ and R₂, together with the nitrogen atom linking them, are a five-membered or six-membered heterocyclic radical, V is hydrogen, halogen, C₁-C₆alkyl, C₁-C₁₂alkoxy, C₁-C₁₂acyloxy, benzyl, phenyl, benzyloxy, phenyloxy, benzyl or benzyloxy each of which is substituted by halogen, cyano, C₁-C₆alkyl or C₁-C₆alkoxy, or V is the group -NT₁T₂ in which T₁ and T₂ independently of one another are each hydrogen, C₁-C₆alkyl, C₅-C₁₀cycloalkyl, benzyl which is unsubstituted or substituted by halogen, cyano, C₁-C₆alkyl or C₁-C₆alkoxy, or acyl having 1 to 8 carbon atoms, and T₁ is also phenyl which is unsubstituted or substituted by halogen, cyano, C₁-C₆alkyl or C₁-C₆alkoxy, and
m is 1 or 2.

6. A phthalide according to any one of claims 1 to 5, wherein X in formula (1) is hydrogen, an N-C₁-C₈alkyl-2-methylindol-3-yl radical, an N-C₁-C₈alkyl-2-phenylindol-3-yl radical or a substituted phenyl radical of the formula (1a) or (1b).

7. A phthalide according to any one of claims 1 to 6, wherein R in formula (1) is C₁-C₆alkyl or phenyl.

8. A phthalide according to any one of claims 1 to 6, wherein R in formula (1), together with R', is C₂-C₃alkylene which is unsubstituted or substituted by methyl.

9. A phthalide according to claim 1, which has the formula 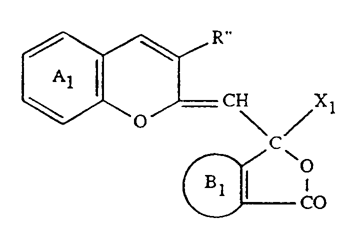 in which
the ring A₁ is unsubstituted or substituted by halogen, C₁-C₆alkyl or C₁-C₆alkoxy and the ring B₁ is a benzene or pyridine ring which is unsubstituted or substituted by halogen, cyano, C₁-C₆alkyl, C₁-C₆alkoxy, C₁-C₆alkylcarbonylamino or di-C₁-C₆alkylamino,
R'' is C₁-C₆alkyl, phenyl or phenyl which is substituted by halogen, C₁-C₆alkyl or C₁-C₆alkoxy,
X₁ is hydrogen, a 3-indolyl radical of the formula 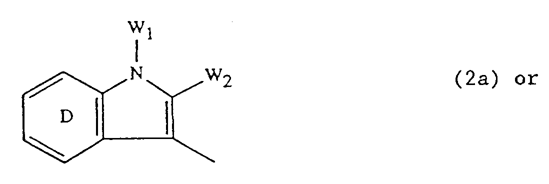 a phenyl radical of the formula 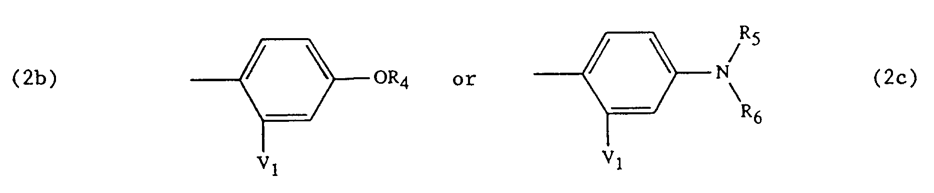 in which W₁ is hydrogen, C₁-C₈alkyl which is unsubstituted or substituted by cyano or C₁-C₆alkoxy, or is acetyl, propionyl or benzyl, W₂ is hydrogen, C₁-C₆alkyl or phenyl,
R₄, R₅ and R₆ independently of one another are each alkyl which has not more than 12 carbon atoms and is unsubstituted or substituted by hydroxyl, cyano or C₁-C₆alkoxy, or are C₅-C₆cycloalkyl, benzyl, phenethyl or phenyl, or (R₅ and R₆), together with the nitrogen atom linking them, are pyrrolidino, piperidino or morpholino, V₁ is hydrogen, halogen, C₁-C₆alkyl, C₁-C₈alkoxy, benzyloxy or the group -NT₃T₄,
T₃ and T₄ independently of one another are each hydrogen, C₁-C₆alkyl, C₁-C₆alkylcarbonyl, or benzoyl which is unsubstituted or substituted by halogen, methyl or methoxy, and the ring D is unsubstituted or substituted by halogen, C₁-C₆alkyl or di-C₁-C₆alkylamino.

10. A phthalide according to claim 9, wherein X₁ in formula (2) is hydrogen, a 3-indolyl radical of the formula (2a) in which W₁ is C₁-C₈alkyl and W₂ is methyl or phenyl, or
a phenyl radical of the formula (2b) in which R₄ is C₁-C₆alkyl and V₁ is hydrogen, and the ring B₁ is a benzene ring which is unsubstituted or substituted by C₁-C₆alkoxy or di-C₁-C₆alkylamino.

11. A phthalide according to claim 1, which has the formula 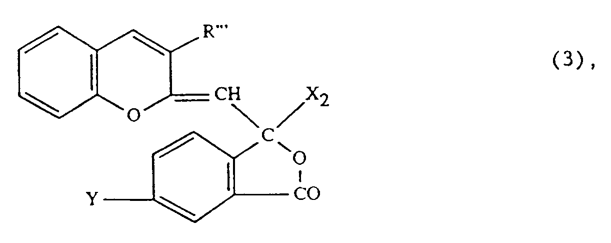 in which
R''' is C₁-C₆alkyl or phenyl,
X₂ is hydrogen, 4-di-C₁-C₆alkylaminophenyl, 4-C₁-C₆alkoxyphenyl or 1-C₁-C₈alkyl-2-methylindol-3-yl and
Y is hydrogen, C₁-C₆alkoxy or di-C₁-C₆alkylamino.

12. A phthalide according to claim 11, wherein Y in formula (3) is dimethylamino.

13. A process for the preparation of a pyran-containing phthalide according to claim 1, which comprises reacting a pyrylium salt of the formula (4a) or a 1,2,3,4-tetrahydroxanthylium salt of the formula (4b) 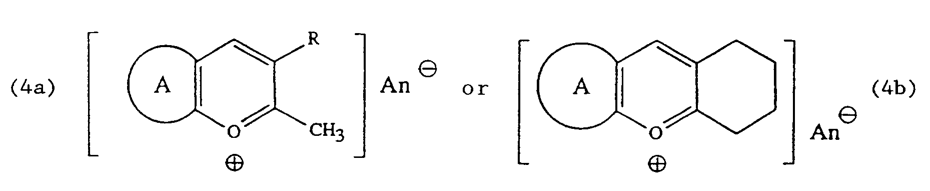 in which A and R are as defined in claim 1 and An^{⊖} is an anion, with an aldehyde-acid or keto-acid of the formula 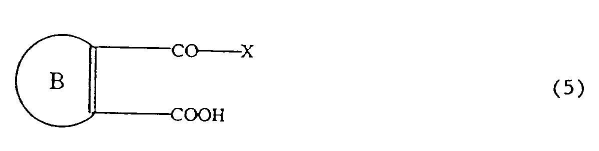 in which X and B are as defined in claim 1.

14. A pressure-sensitive or heat-sensitive recording material which contains at least one phthalide compound according to any one of claims 1 to 12 as the colour-former in its colour reactant system.

15. A pressure-sensitive recording material according to claim 14, which comprises the phthalide compound, dissolved in an organic solvent, and at least one solid electron acceptor.

16. A pressure-sensitive recording material according to either claim 14 or claim 15, wherein the phthalide compound is encapsulated in microcapsules.

17. A pressure-sensitive recording material according to claim 16, wherein the encapsulated phthalide compound is present in the form of a layer on the reverse side of a transfer sheet, and the electron acceptor is present in the form of a layer on the front side of the receiver sheet.

18. A heat-sensitive recording material according to claim 14, which comprises, in at least one layer, at least one phthalide compound according to any one of claims 1 to 14, an electron acceptor and, if appropriate, a binder and/or wax.

19. A recording material according to any one of claims 14 to 18, wherein the phthalide compound is present together with one or more other colour-formers.

## Revendications

1. Phtalides contenant un groupe pyranne de formule 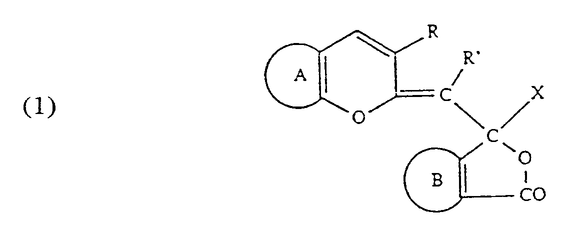 dans laquelle
R représente un atome d'hydrogène, groupe alkyle comportant au plus 12 atomes de carbone, non substitué ou substitué par un atome d'halogène, un groupe cyano, hydroxy ou alcoxy en C₁₋₆, un groupe cycloalkyle, un groupe phényle ou phénéthyle non substitué ou portant un substituant halogéno, cyano, méthyle, trifluorométhyle, méthoxy ou carbométhoxy, un groupe phénylisopropyle, un groupe benzyle ou un résidu hétérocyclique à 5 ou 6 chaînons à caractère aromatique,
R' représente un atome d'hydrogène ou
R et R' représentent ensemble un radical alkylène en C₂₋₃ éventuellement substitué par un méthyle,
X représente un atome d'hydrogène, un groupe alkyle comportant de 1 à 12 atomes de carbone, ou un groupe phényle ou naphtyle non substitué ou portant un substituant halogéno, cyano, alkyle en C₁₋₆ cycloalkyle en C₅₋₆, acyle en C₁₋₈, -NR₁R₂, -OR₃ ou -SR₃, où R₁, R₂ et R₃ représentent indépendamment un atome d'hydrogène, un groupe alkyle comportant au plus 12 atomes de carbone substitué ou non par un atome d'halogène, un groupe hydroxy, cyano ou alcoxy en C₁₋₆, un groupe acyle comportant de 1 à 8 atomes de carbone, un groupe cycloalkyle comportant de 5 à 10 atomes de carbone, ou un groupe phényle ou phénéthyle substitué ou non par des résidus halogéno, trifluorométhyle, cyano, alkyle en C₁₋₆, alcoxy en C₁₋₆, (alcoxy en C₁₋₆)-carbonyle, -NX'X'' ou X'X''N-phénylamino, où X' et X'' représentent indépendamment un atome d'hydrogène, un groupe alkyle en C₁₋₆, cyclohexyle, benzyle ou phényle, ou R₁ et R₂ représentent ensemble avec l'atome d'azote les reliant, un résidu pyrrolidino, pipéridino, pipécolino, morpholino, thiomorpholino, pipérazino, N-méthylpipérazino, N-phénylpipérazino ou N-alkylimidazolino, ou
X représente un groupe thiényle, acridinyle, benzofuranyle, benzothiényle, naphtothiényle, phénothiazinyle, pyrrolyle, indolyle, carbazolyle, julolidinyle, kaïrolinyle, indolinyle, dihydroquinoléinyle ou tétrahydroquinoléinyle, non substitué ou portant sur un atome de carbone, des substituants halogéno, hydroxy, cyano, nitro, alkyle en C₁₋₆, alcoxy en C₁₋₆, alkylthio en C₁₋₆, (alcoxy en C₁₋₆)-carbonyle, acyle en C₁₋₈, amino, alkylamino en C₁₋₆, (alkyle en C₁₋₆)-carbonylamino ou di-(alkyle en C₁₋₆)-amino, cycloalkyle en C₅₋₆, benzyle ou phényle, ou un groupe acridinyle, phénothiazinyle, pyrrolyle, indolyle, carbazolyle, julolidinyle, kaïrolinyle, indolinyle, dihydroquinoléinyle ou tétrahydroquinoléinyle, substitué sur un atome d'azote par un groupe alkyle en C₁₋₁₂, alcényle en C₂₋₁₂, cycloalkyle en C₅₋₁₀, acyle en C₁₋₈, phényle, benzyle, phénéthyle ou phénisopropyle, et
Le noyau A représente un noyau benzène ou naphtalène éventuellement substitué par un ou plusieurs atomes d'halogène ou groupes alkyle en C₁₋₆, alcoxy en C₁₋₆ ou (alcoxy en C₁₋₆)-carbonyle, et
le noyau B représente un noyau benzène substitué ou non par un ou plusieurs atomes d'halogène, groupes cyano, nitro, alkyle en C₁₋₆, alcoxy en C₁₋₆, alkylthio en C₁₋₆, (alkyle en C₁₋₆)-carbonyle, (alcoxy en C₁₋₆)-carbonyle, amino, (alkyle en C₁₋₆)-amino, di-(alkyle en C₁₋₆)-amino ou (alkyle en C₁₋₆)-carbonylamino, ou un noyau pyridine ou pyrazine, ou un noyau naphtalène, quinoléine ou quinoxaline.

2. Phtalides conformes à la revendication 1, caractérisés en ce que, dans la formule (1), le noyau A représente un noyau benzène non substitué ou substitué par un atome d'halogène ou un groupe alkyle en C₁₋₆.

3. Phtalides conformes à une des revendications 1 et 2, caractérisés en ce que le noyau B représente un noyau benzène, naphtalène, pyridine, pyrazine, quinoxaline ou quinoléine pouvant être substitués.

4. Phtalides conformes à une des revendications 1 à 3, caractérisés en ce que, dans la formule (1), le noyau B représente un noyau benzène non substitué ou portant un substituant chloro, alcoxy en C₁₋₆ ou di-(alkyle en C₁₋₆)-amino.

5. Phtalides conformes à une des revendications 1 à 4, caractérisés en ce que, dans la formule (1), X représente un résidu phényle de formule 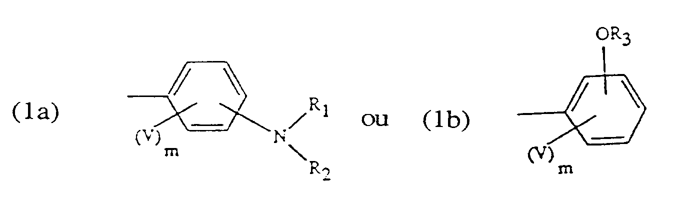 dans lesquelles
R₁, R₂ et R₃ représente indépendamment un atome d'hydrogène, un groupe alkyle comportant au plus 12 atomes de carbone substitué ou non par un atome d'halogène, un groupe hydroxy, cyano ou alcoxy en C₁₋₆, un groupe acyle comportant de 1 à 8 atomes de carbone, un groupe cycloalkyle comportant de 5 à 10 atomes de carbone, ou un groupe phényle ou phénalkyle substitué ou non, sur le cycle, par un atome d'halogène, un groupe trifluorométhyle, cyano, alkyle en C₁₋₆, alcoxy en C₁₋₆, (alcoxy en C₁₋₆)-carbonyle, -NX'X'' ou X'X''N-phénylamino, où X' et X'' représentent indépendamment un atome d'hydrogène, un groupe alkyle en C₁₋₆, cyclohexyle, benzyle ou phényle, ou R₁ et R₂ représentent ensemble avec l'atome d'azote les reliant un résidu hétérocyclique à 5 ou 6 chaînons,
V représente un atome d'hydrogène ou d'halogène, un groupe alkyle C₁₋₆, alcoxy C₁₋₁₂, acyloxy C₁₋₁₂, benzyle, phényle, benzyloxy, phényloxy, benzyle ou benzyloxy portant un substituant halogéno, cyano, alkyle C₁₋₆ ou alcoxy C₁₋₆, ou le groupe -NT₁T₂,T₁ et T₂ représentant indépendamment un atome d'hydrogène, un groupe alkyle C₁₋₆, cycloalkyle C₅₋₁₀, benzyle non substitué ou portant des substituants halogéno, cyano, alkyle C₁₋₆ ou alcoxy C₁₋₆, ou un groupe acyle comportant de 1 à 8 atomes de carbone, T₁ pouvant représenter également un résidu phényle substitué ou non par des résidus halogéno, cyano, alkyle C₁₋₆ ou alcoxy C₁₋₆, et m est égal à 1 ou 2.

6. Phtalides conformes à une des revendications 1 à 5, caractérisés en ce que, dans la formule (1), X représente un atome d'hydrogène, un un groupe N-(alkyle en C₁₋₈)-2-méthylindol-3-yle, N-(alkyl en C₁₋₈)-2-phénylindol-3-yle ou un résidu phényle substitué de formule (1a) ou (1b).

7. Phtalides conformes à une des revendications 1 à 6, caractérisés en ce que, dans la formule (1), R représente un groupe alkyle en C₁₋₆ ou phényle.

8. Phtalides conformes à une des revendications 1 à 6, caractérisés en ce que, dans la formule (1), R en combinaison avec R' représente un groupe alkylène en C₂₋₃ éventuellement substitué par un groupe méthyle.

9. Phtalides conformes à la revendication 1, caractérisés en ce qu'ils correspondent à la formule 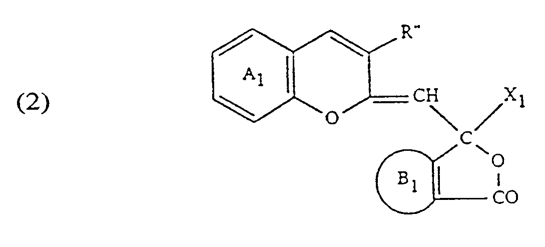 dans laquelle
le noyau A₁ est substitué ou non par un atome d'halogène, un groupe alkyle en C₁₋₆ ou alcoxy en C₁₋₆, et le noyau B₁ est un noyau benzène ou pyridine éventuellement substitué par un résidu halogéno, cyano, alkyle en C₁₋₆, alcoxy en C₁₋₆, (alkyle en C₁₋₆,)-carbonylamino ou di-(alkyle en C₁₋₆,)-amino,
R'' représente un groupe alkyle en C₁₋₆, phényle ou phényle substitué par un groupe alkyle en C₁₋₆, ou alcoxy en C₁₋₆,
X₁ représente un atome d'hydrogène, un résidu 3-indolyle de formule 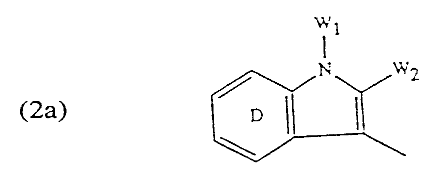 ou un résidu phényle substitué de formule 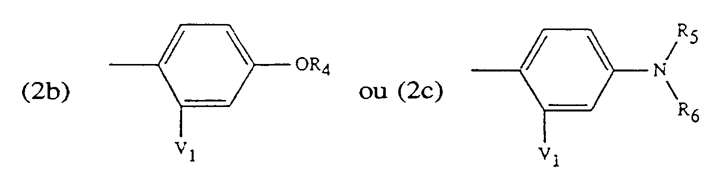 dans laquelle W₁ représente un atome d'hydrogène, un groupe acétyle, propionyle, benzyle ou (alkyle en C₁₋₈) substitués ou non par un groupe cyano ou alcoxy en C₁₋₆.
R₄, R₅ et R₆ représentent indépendamment un groupe alkyle comportant au plus 12 atomes de carbone substitué ou non par un hydroxy, cyano ou alcoxy en C₁₋₆, un groupe cycloalkyle en C₅₋₆, benzyle, phénéthyle ou phényle, ou (R₅ et R₆) forment ensemble avec l'atome d'azote les reliant un noyau pyrrolidino, pipéridino ou morpholino,
V₁ représente un atome d'hydrogène ou d'halogène, un groupe alkyle en C₁₋₆, alcoxy en C₁₋₈, benzyloxy ou le groupe -NT₃T₄, T₃ et T₄ représentant indépendamment un atome d'hydrogène, un groupe alkyle en C₁₋₆, (alkyle en C₁₋₆)-carbonyle ou benzoyle substitué ou non par un résidu halogéno, méthyle ou méthoxy, et le noyau D est substitué ou non par un résidu halogéno, alkyle en C₁₋₆ ou di-(alkyle en C₁₋₆)-amino.

10. Phtalides conformes à la revendication 9, caractérisés en ce que; dans la formule (2), X₁ représente un atome d'hydrogène, un résidu 3-indolyle de formule (2a), où W₁ est un résidu alkyle en C₁₋₈, W₂ est un méthyle ou phényle, ou un résidu phényle de formule (2b), où R₄ est un résidu alkyle en C₁₋₆ et V₁ un atome d'hydrogène, et le noyau B₁ représente un noyau benzène éventuellement substitué par un alcoxy en C₁₋₆ ou di(alkyle en C₁₋₆)-amino.

11. Phtalides conformes à la revendication 1, caractérisés en ce qu'ils correspondent à la formule 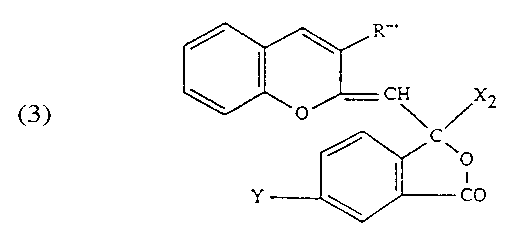 dans laquelle
R''' est un groupe alkyle en C₁₋₆,
X₂ est un atome d'hydrogène, un groupe 4-di-(alkyle en C₁₋₆)-aminophényle, 4-(alcoxy en C₁₋₆)-phényle ou 1-(alkyle en C₁₋₈)-2-méthylindol-3-yle, et
Y est un atome d'hydrogène, un groupe alcoxy en C₁₋₆ ou di(alkyle en C₁₋₆)-amino.

12. Phtalides conformes à la revendication 11, caractérisés en ce que, dans la formule (3), Y représente un groupe diméthylamino.

13. Procédé pour la préparation de phtalides pyranniques conformes à la revendication 1, caractérisé en ce que l'on fait réagir un sel de pyrylium de formule (4a) ou un sel de 1,2,3,4-tétrahydro-xanthylium de formule (4b) 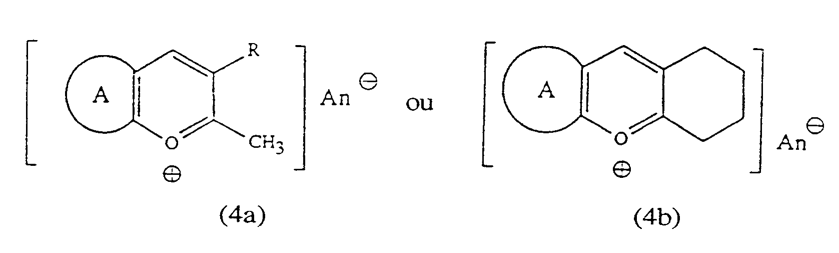 dans lesquelles A et R ont la signification indiquée dans la revendication 1, et A⁻ représente un anion,
avec un acide-aldéhyde ou acide-cétone de formule 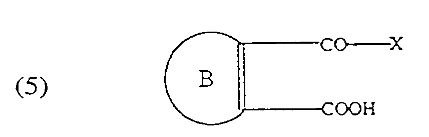 dans laquelle X et B ont la signification indiquée dans la revendication 1.

14. Matériau d'enregistrement sensible à la pression ou à la chaleur, caractérisé en ce qu'il contient, dans son système de réactifs chromogènes, comme agent chromogène au moins un des composés de type phtalide conformes à une des revendications 1 à 12.

15. Matériau d'enregistrement sensible à la pression, caractérisé en ce qu'il contient les composés de type phtalide, dissous dans un solvant organique, et au moins un accepteur d'électrons solide.

16. Matériau d'enregistrement sensible à la pression conforme à une des revendications 14 et 15, caractérisé en ce que les composés de type phtalide sont encapsulés dans des microcapsules.

17. Matériau d'enregistrement sensible à la pression conforme à la revendication 16, caractérisé en ce que les composés de type phtalide encapsulés sont sous forme d'une couche sur le côté verso d'une feuille de transfert et l'accepteur d'électrons est sous forme d'une couche sur le côté recto d'une feuille réceptrice.

18. Matériau d'enregistrement thermosensible conforme à la revendication 14, caractérisé en ce qu'il comprend, dans au moins une couche, au moins un des phtalides conformes à une des revendications 1 à 14, un accepteur d'électrons et éventuellement un liant et/ou une cire.

19. Matériau d'enregistrement conforme à une des revendications 14 à 18, caractérisé en ce que les composés de type phtalide sont présents en combinaison avec un ou plusieurs agents chromogènes conventionnels.
